# EUROPÄISCHE PATENTSCHRIFT

(11) **EP 3 717 888 B1**
(45) Veröffentlichungstag und Bekanntmachung des Hinweises auf die Patenterteilung: **14.12.2022**
(21) Anmeldenummer: 18822271.5
(22) Anmeldetag: 27.11.2018
(51) Int. Cl.: G01N 15/14, G01N 1/31, G01N 1/38, G01N 15/00, G01N 15/10

(54) **DURCHFLUSSZYTOMETERANORDNUNG**
FLOW CYTOMETER ARRANGEMENT
ENSEMBLE CYTOMÈTRE EN FLUX

(30) Priorität: 27.11.2017 DE 102017128029
(43) Veröffentlichungstag der Anmeldung: 07.10.2020
(73) Patentinhaber: Kuhn, Martin, 8192 Glattfelden (CH)
(72) Erfinder: Kuhn, Martin, 8192 Glattfelden (CH)
(74) Vertreter: Schlosser, Martin
(86) Internationale Anmeldenummer: PCT/EP2018/082712
(87) Internationale Veröffentlichungsnummer: WO 2019/102038

(56) Entgegenhaltungen:
- EP-A1- 0 634 640
- WO-A1-97/07390
- US-A1- 2014 087 389

## Beschreibung

Die Erfindung betrifft eine Durchflussztometeranordnun.

Das Dokument WO 97/07390 A1 offenbart ein Verfahren und eine Vorrichtung zum Bestimmen der Anzahl von Partikeln oder Zellen in einer Flüssigkeitsprobe. Die Vorrichtung beinhaltet ein Flüssigkeitssystem, welches erlaubt, eine Messsequenz zu unterbrechen, sobald Berechnungsmittel anzeigen, dass die Unsicherheit einer aktuell gemessenen Anzahl an Zellen klein genug ist, um der Messung zu vertrauen. Die Vorrichtung ist dann bereit, mit der nächsten Probe fortzufahren.

Der hygienische Zustand des Trinkwassers wird seit über 100 Jahren mit der sogenannten AMK-Methode (AMK = aerobe, mesophile Keime) ermittelt. Dabei werden typischerweise Wasserproben auf einen Nährboden aufgebracht und über beispielsweise 72 Stunden bebrütet (inkubiert). Danach werden die möglicherweise entstandenen Kolonien gezählt, was einen Rückschluss auf die Anzahl der im Trinkwasser vorhandenen kolonienbildenden Einheiten (KBE) zulässt.

Diese herkömmliche Methode ist sehr ungenau, wobei lediglich die aeroben, mesophilen Keime erfasst werden. Sie ist des Weiteren zeitintensiv und kann nur in dafür spezialisierten Labors durchgeführt werden.

Das Trinkwasser ist heutzutage immer größeren Einflüssen ausgesetzt, beispielsweise Temperaturanstieg, Extremwettersituationen oder Belastungen durch Landwirtschaft und Industrie. Um bei einem relevanten Vorkommnis umgehend Maßnahmen treffen zu können, sind neue Technologien zur zeitnahen Bestimmung der mikrobiologischen Situation erforderlich.

Sedimente sowie organische und anorganische Parameter im Trinkwasser können bereits jetzt online überwacht werden. Zur Messung der mikrobiologischen Parameter vor Ort fehlt jedoch die Technologie bis heute.

Die bereits weiter oben erwähnte Zahl aerober, mesophiler Keime (AMK) wird heutzutage als Maß für die mikrobielle Verunreinigung durch Bakterien, Hefen und Schimmelpilze eines Lebensmittels angesehen. Durch Bebrüten der bereits erwähnten Wasserprobe auf Nährböden wachsen aerobe, mesophile Keime, wenn vorhanden, zu Kolonien.

Es hat sich jedoch herausgestellt, dass diese Methode nur einen geringen Teil von etwa 0,01 % bis 1 % der im Wasser vorhandenen Mikroorganismen erfasst. Die in der Schweizer Hygieneverordnung festgelegten AMK-Toleranzwerte von 20 KBE/ml im behandelten Trinkwasser und 300 KBE/ml im Verteilnetz repräsentieren die wirklich vorhandenen, aktiven Zellen bei Weitem nicht.

Abgesehen von den bereits erwähnten Nachteilen wie der erheblich zu geringen ermittelten Zellenzahl und der sehr langsamen Durchführung von beispielsweise 48 bis 72 Stunden ist noch anzumerken, dass Toleranz- und Grenzwerte je nach Nährmedium und Bebrütungstechnologie schwanken.

Dabei kann von einer Korrelation zwischen AMK und Gesamtzellzahl nicht ausgegangen werden, da der Anteil an kultivierbaren Zellen stark variiert.

Um die tatsächliche Anzahl von Zellen im Trinkwasser zu ermitteln, wurde bereits vorgeschlagen, die Durchflusszytometrie einzusetzen. Dabei werden Wasserproben typischerweise mit Farbstoffen vermischt, welche an die DNA oder RNA von Bakterien binden und diese somit sichtbar machen. Die derart markierten Bakterien können dann erfasst und einzeln gezählt werden, wobei beispielsweise eine Anregung mittels eines Laserstrahls erfolgen kann.

Beim Trinkwasser der Stadt Zürich erhält man mittels der weiter oben erwähnten AMK-Methode beispielsweise zwischen 0 und 10 Zellen pro Milliliter. Mit der Durchflusszytometrie wird hingegen eine Gesamtzellzahl zwischen 80.000 und 100.000 Zellen pro Milliliter ermittelt. Derart große Zahlen setzen ein Umdenken voraus, seitens der Wasserversorger und auch seitens der Konsumenten. In Wasserproben aus der Umwelt beträgt der Anteil an intakten Zellen meist etwa 80 % bis 90 %, wie eine Vielzahl von mikroskopischen Untersuchungen belegen. Es kann angenommen werden, dass durch eine Bestimmung der Gesamtzellzahl mittels Durchflusszytometrie die tatsächliche Zahl an Mikroorganismen im Trinkwasser zuverlässig bestimmbar ist.

An der mikrobiologischen Wasserqualität ändert sich jedoch nichts, wenn mit der Durchflusszytometrie deutlich höhere Zellzahlen bestimmt werden.

Die Durchflusszytometrie ist dabei seit etwa 50 Jahren aus der Medizintechnik bekannt. In Kliniken und medizinischen Labors wird sie unter anderem in der Hämatologie, Infektologie und Immunologie zur Routinediagnostik eingesetzt. Weitere wichtige Einsatzgebiete sind medizinische und zellbiologische Grundlagenforschung und die quantitative Untersuchung von Zellen in der Biologie. Durchflusszytometer können auch die Gesamtzellzahl (GZZ), das Verhältnis von HNA (High Nucleic Acid) und LNA (Low Nucleic Acid) sowie von lebenden und toten Zellen zählen. Die Mikroorganismen und Partikel werden dabei nach Anfärben und Inkubieren einzeln durch eine Glaskapillare geschleust und mit einem fokussierten Laserstrahl beleuchtet, um den DNA-Farbstoff anzuregen. Trifft der Laser auf eine Zelle oder ein Partikel, wird das gestreute Licht im Streulichtkanal erfasst und das emittierte Licht der Zellen über die Fluoreszenzkanäle detektiert.

Das Verfahren der Durchflusszytometrie ist bislang im Wesentlichen aus unterschiedlichen medizinischen Gebieten bekannt. Dabei wird grundsätzlich davon ausgegangen, dass das Verfahren in einem Labor von Fachpersonal durchgeführt wird. Hierzu muss eine Probe manuell zugeführt werden. Außerdem sind die im Markt befindlichen Geräte für Laborbedingungen spezifiziert, weshalb sie beispielsweise empfindlich auf Umwelteinflüsse wie Luftfeuchtigkeit und Temperatur reagieren. Flüssigkeitsbehälter für diverse Betriebsflüssigkeiten werden typischerweise nicht mit Sensoren überwacht, sondern werden nur optisch überprüft. Der Betrieb solcher Anlagen ist nicht kontinuierlich möglich. Außerdem sind keine elektrischen Schnittstellen vorhanden, um mit weiteren Geräten oder Systemen zu kommunizieren. Bekannte, im Markt befindliche Durchflusszytometer eigenen sich somit nicht für die Überwachung des Trinkwassers in einem typischen städtischen oder kommunalen Wasserversorgungssystem, da dies einen automatisierten Betrieb ohne ständige Überwachung durch qualifiziertes Personal erfordert. Außerdem wäre es wünschenswert, die Toleranz gegen Umwelteinflüsse zu erhöhen, um die Überwachung der Trinkwasserqualität unter den typischen Bedingungen einer solchen Wasserversorgung zu ermöglichen, welche sich von den planbaren und konstanten Laborbedingungen deutlich unterscheiden.

Dies wird erfindungsgemäß durch eine Durchflusszytometeranordnung nach Anspruch 1 erreicht. Vorteilhafte Ausgestaltungen können beispielsweise den Unteransprüchen entnommen werden. Der Inhalt der Ansprüche wird durch ausdrückliche Bezugnahme zum Inhalt der Beschreibung gemacht.

Die Erfindung betrifft eine Durchflusszytometeranordnung. Die Durchflusszytometeranordnung weist eine Durchflussmesszelle und einen Mischer auf. Sie weist eine erste Pumpe und eine zweite Pumpe auf. Sie weist einen Eingangsanschluss, ein Farbstoffreservoir und ein Hüllflüssigkeitsreservoir auf.

Die erste Pumpe ist dabei eingangsseitig mit dem Eingangsanschluss zum Ansaugen von Probenflüssigkeit verbunden. Die zweite Pumpe ist eingangsseitig mit dem Farbstoffreservoir zum Ansaugen von Farbstoff verbunden.

Die erste Pumpe und die zweite Pumpe sind ausgangsseitig mit dem Mischer verbunden, um Probenflüssigkeit und Farbstoff in den Mischer zu pumpen. Der Mischer ist zum Mischen der Probenflüssigkeit und des Farbstoffs zu einem Gemisch ausgebildet.

Die zweite Pumpe ist eingangsseitig mit dem Mischer zum Ansaugen des Gemisches verbunden. Diese Verbindung kann dabei beispielsweise direkt oder indirekt erfolgen. Eine indirekte Verbindung kann beispielsweise über einen weiter unten erwähnten Inkubator erfolgen.

Die erste Pumpe ist eingangsseitig mit dem Hüllflüssigkeitsreservoir zum Ansaugen von Hüllflüssigkeit verbunden.

Die zweite Pumpe ist ausgangsseitig mit der Durchflussmesszelle verbunden, um aus dem Gemisch einen Probenstrahl durch die Durchflussmesszelle zu erzeugen. Die erste Pumpe ist ausgangsseitig mit der Durchflussmesszelle verbunden, um aus der Hüllflüssigkeit einen Hüllstrahl durch die Durchflussmesszelle zu erzeugen, welcher den Probenstrahl umgibt.

Die erfindungsgemäße Durchflusszytometeranordnung erlaubt eine automatisierte Durchführung der Durchflusszytometrie, was einen ständigen manuellen Eingriff durch Fachpersonal oder auch nur eine Beaufsichtigung entbehrlich werden lässt. Die erfindungsgemäße Durchflusszytometeranordnung ist dabei so ausgebildet, dass alle für eine erfolgreiche Messung von Zellen in Proben notwendigen Schritte vollautomatisiert durchgeführt werden können. Dadurch kann auch auf unterschiedliche Umgebungsbedingungen besser eingegangen werden, da eine automatisierte Überwachung nach Bedarf möglich ist.

Durch die Ausführung mit nur zwei Pumpen ist die beschriebene Durchflusszytometeranordnung sehr kompakt.

Der Probenstrahl durch die Durchflussmesszelle ist typischerweise derjenige Strahl, in welchem die zu messenden Zellen markiert sind und einzeln vorliegen, so dass sie durch geeignete, weiter unten beschriebene Vorgehensweisen gemessen und/oder gezählt werden können.

Der Mischer dient typischerweise dazu, Probenflüssigkeit mit Farbstoff zu vermischen, um den bereits weiter oben beschriebenen Einbau von Farbstoffen in eine DNA oder RNA von Zellen zu ermöglichen. Es sei verstanden, dass es sich bei dem Farbstoffreservoir und dem Hüllflüssigkeitsreservoir insbesondere um Container handeln kann, welche Teil der Durchflusszytometeranordnung sein können. Es kann sich bei dem Farbstoffreservoir und/oder bei dem Hüllflüssigkeitsreservoir jedoch beispielsweise auch um Anschlüsse an eine externe Versorgung mit Farbstoff und/oder Hüllflüssigkeit handeln.

Die Hüllflüssigkeit erzeugt wie erwähnt einen Hüllstrahl, welcher typischerweise zur hydrodynamischen Fokussierung des Probenstrahls dient. Dadurch kann der Probenstrahl beispielsweise von einer ursprünglichen Ausdehnung von beispielsweise 90 µm bis 110 µm, insbesondere 100 µm, auf eine Ausdehnung von beispielsweise 20 µm bis 40 µm, insbesondere 30 µm, verkleinert werden. Die Ausdehnung bezieht sich dabei insbesondere auf einen Durchmesser des Probenstrahls in der Durchflussmesszelle. Durch diese Verkleinerung wird ein geeigneter Abstand der zu detektierenden Zellen eingestellt.

Gemäß einer Weiterbildung weist die Durchflusszytometeranordnung ferner einen weiteren Mischer auf. Dieser kann insbesondere eingangsseitig mit der ersten Pumpe und mit der zweiten Pumpe verbunden sein. Ausgangsseitig kann der Mischer mit der zweiten Pumpe verbunden sein, wobei es sich auch hierbei um eine direkte oder eine indirekte Verbindung, insbesondere über einen Inkubator, handeln kann.

Die jeweiligen Verbindungen zwischen den Pumpen, also der ersten Pumpe und der zweiten Pumpe, und den Mischern sind dabei insbesondere schaltbar ausgeführt, so dass der Mischer und der weitere Mischer alternativ verwendbar sind. Dies kann insbesondere bedeuten, dass durch eine geeignete Anordnung von Ventilen festgelegt werden kann, ob die erste Pumpe und die zweite Pumpe ausgangsseitig mit dem Mischer oder mit dem weiteren Mischer verbunden werden, so dass beide Mischer zum entsprechenden Mischen von Probenflüssigkeit mit Farbstoff verwendet werden können. Insbesondere kann auch schaltbar sein, ob der Mischer oder der weitere Mischer ein jeweiliges Gemisch zu der zweiten Pumpe liefern soll.

Bevorzugt ist dabei ein jeweiliger Mischer ausgangsseitig mit einem jeweiligen Inkubator verbunden, welcher wiederum ausgangsseitig mit der zweiten Pumpe verbunden ist. Der Inkubator dient insbesondere dazu, für die nötigen Rahmenbedingungen zu sorgen, um den Farbstoff zuverlässig in die Zellen einzubauen bzw. an DNA oder RNA anzudocken.

Es sei verstanden, dass sowohl der Mischer wie auch der weitere Mischer, sofern vorhanden, jeweils mit einem Inkubator verbunden sein können, wobei auch nur entweder der Mischer oder der weitere Mischer mit einem Inkubator verbunden sein kann oder auch kein Inkubator vorhanden sein kann.

Ein jeweiliger Inkubator kann insbesondere eine jeweilige Heizeinrichtung aufweisen, um ein aus dem Mischer in den Inkubator abgegebenes Gemisch für einen vorgegebenen Zeitraum auf eine vorgegebene Temperatur zu heizen. Beispielsweise kann ein solcher Inkubator dafür ausgebildet sein, ein Gemisch für einen Zeitraum von 5 Minuten auf eine Temperatur zwischen 35 °C und 45 °C, insbesondere 40 °C, zu heizen. Dies sind typische Rahmenbedingungen für einen zuverlässigen Einbau von Farbstoffmolekülen in zu detektierende Zellen bzw. für ein Andocken an die Zellen.

Gemäß einer bevorzugten Ausführung ist die erste Pumpe dazu ausgebildet, ein vorgegebenes erstes Volumen an Probenflüssigkeit von dem Eingangsanschluss anzusaugen. Weiter bevorzugt ist die zweite Pumpe dazu ausgebildet, ein vorgegebenes zweites Volumen an Farbstoff von dem Farbstoffreservoir anzusaugen. Noch weiter bevorzugt sind die erste Pumpe und die zweite Pumpe dazu ausgebildet, das erste Volumen an Probenflüssigkeit und das zweite Volumen an Farbstoff, zeitlich dem Ansaugen nachgelagert, gleichzeitig in den Mischer zu pumpen.

Dadurch kann ein diskontinuierlicher Betrieb gewährleistet werden, wobei zunächst Probenflüssigkeit in die erste Pumpe und Farbstoff in die zweite Pumpe gepumpt wird und Probenflüssigkeit und Farbstoff anschließend in definierter Weise in den Mischer abgegeben werden. Durch die Gleichzeitigkeit der Abgabe in den Mischer wird eine besonders gute Vermischung sichergestellt. Dabei können insbesondere auch definierte Volumina bzw. Volumenverhältnisse zwischen Probenflüssigkeit und Farbstoff in den Mischer abgegeben werden. Es sei verstanden, dass es sich bei dem Mischer auch um den weiteren Mischer handeln kann. Dies gilt für entsprechende Vorkommnisse des Begriffs des Mischers in dieser Anmeldung.

Die Pumpen können insbesondere als spindelbetätigte Kolbenpumpen ausgeführt sein. Dies hat sich in der Praxis bewährt, da die erforderliche Genauigkeit bei der Definition von Volumina und Drücken damit erreicht werden kann.

Gemäß einer bevorzugten Ausführung weist die Durchflusszytometeranordnung eine Detektoranordnung auf. Die Detektoranordnung weist wiederum einen Laser auf, welcher dazu ausgebildet ist, einen Laserstrahl zu erzeugen. Außerdem weist sie eine Richtoptik auf, welche dazu ausgebildet ist, den Laserstrahl auf die Durchflussmesszelle zu richten. Des Weiteren weist sie eine Anzahl von Detektoren auf, welche dazu ausgebildet sind, den durch die Durchflussmesszelle hindurchgetretenen Laserstrahl zu detektieren.

Eine solche Detektoranordnung ermöglicht insbesondere eine vollautomatisierte Detektion mit hoher Genauigkeit der gesuchten Zellen in der Probenflüssigkeit.

Unter einer Anzahl sei dabei eine Zahl von einem oder auch mehreren verstanden. Es kann sich also zum Beispiel um einen Detektor, zwei Detektoren, drei Detektoren, vier Detektoren oder auch noch mehr Detektoren handeln.

Der Laser kann beispielsweise dazu ausgebildet sein, einen Laserstrahl mit einer Wellenlänge von 488 nm zu erzeugen. Dies hat sich für typische Farbstoffe bewährt. Auch andere Wellenlängen bzw. Spektralbereiche können jedoch verwendet werden.

Bevorzugt weist die Detektoranordnung ein zumindest teilweise umgebendes, wannenförmiges Gehäuse auf, welches einstückig ausgebildet ist. Es kann auch davon gesprochen werden, dass das Gehäuse als Monoblock ausgebildet ist. Dies kann insbesondere bedeuten, dass das Gehäuse aus einem einzigen Block hergestellt wurde, welcher insbesondere durch spanabhebende Verfahren wie beispielsweise Fräsen in seine endgültige Form gebracht wurde.

Durch diese einstückige Ausführung wird eine besonders hohe Stabilität erreicht, wobei sich gezeigt hat, dass dies eine besonders vorteilhafte Stabilität der enthaltenen Komponenten relativ zueinander erzeugt. Dadurch wird eine zuverlässige Messung auch unter schwierigen Bedingungen wie beispielsweise Temperaturschwankungen und Erschütterungen sichergestellt. Der Einsatz der Durchflusszytometeranordnung außerhalb von definierten Laborbedingungen wird dadurch erheblich erleichtert und die Zuverlässigkeit wird erhöht.

Das Gehäuse kann insbesondere eine Anzahl von Unterstützungsvorsprüngen für Komponenten der Detektoranordnung aufweisen. Derartige Unterstützungsvorsprünge können beispielsweise als Erhebungen in einem Boden des Gehäuses ausgebildet sein.

Das Gehäuse kann beispielsweise durch einen Deckel zu einem vollständig umschließenden Gehäuse gemacht werden. Ein solches vollständig umschließendes Gehäuse kann beispielsweise einen vollständigen Schutz gegen Lichteinfall und gegen das Eindringen von Verschmutzungen und/oder Flüssigkeiten bieten.

Das Gehäuse kann beispielsweise aus Aluminium ausgebildet sein. Auch andere Materialien, insbesondere andere Metalle, können jedoch verwendet werden.

Bevorzugt weist die Detektoranordnung eine Mehrzahl von Detektoren auf, welche unterschiedliche Spektralbereiche detektieren. Beispielsweise können vier Detektoren verwendet werden, welche entsprechend das blaue, das rote, das grüne und das gelbe Spektrum abdecken können. Dies hat sich für die Praxis als vorteilhaft erwiesen, da Spektralbereiche ausgewertet werden können, mittels welchen die gesuchten Zellen zuverlässig detektiert werden können.

Es sei verstanden, dass das erwähnte, als Monoblock bzw. einstückig ausgeführte Gehäuse, insbesondere einer Detektoranordnung, auch als eigenständiger Erfindungsaspekt angesehen werden kann.

Gemäß einer bevorzugten Ausführung weist die Detektoranordnung eine Doppelspalt-Streulichtblende auf, wobei ein gestreuter Strahl des Laserstrahls zur Unterdrückung eines Signal-Offsets auf einen Steg der Doppelspalt-Streulichtblende gerichtet ist. Auch eine solche Doppelspalt-Streulichtblende kann als eigenständiger Erfindungsaspekt aufgefasst werden.

Mittels der Doppelspalt-Streulichtblende ist es möglich, den ursprünglich auf den Probenstrahl gerichteten Laserstrahl auszublenden, so dass Streulicht, welches tatsächlich durch in der Probenflüssigkeit enthaltene Zellen oder Partikel ausgelöst wird, von Streulicht getrennt werden kann, welches durch reine Streuung des Ursprungsstrahls an Komponenten der Durchflussmesszelle, insbesondere eines Röhrchens, entstanden ist. Somit kann auch die entsprechende Wellenlänge des ursprünglich anregenden Lasers gemessen und zur Auswertung verwendet werden.

Bei dem Laser kann es sich insbesondere um einen Diodenlaser handeln. Dies hat sich für typische Anwendungen als vorteilhaft erwiesen, da es sich um eine zuverlässige und langlebige Ausführung eines Lasers handelt.

Der Laser kann insbesondere Teil einer Lasereinheit sein, welche ferner eine lichtleitende Faser zum Einkoppeln des Laserstrahls sowie eine Einkopplungseinheit aufweist. Die Einkopplungseinheit kann dabei ein dem Laser zugewandtes Ende der lichtleitenden Faser halten. Dies erlaubt ein einfaches Einkoppeln des aus dem Laser emittierten Lichts in die lichtleitende Faser.

Die Einkopplungseinheit ist dabei dazu ausgebildet, eine Position des gehaltenen Endes der lichtleitenden Faser relativ zum Laser verstellbar zu positionieren. Dabei kann insbesondere eine Verstellbarkeit in x-y-Richtung, also insbesondere in einer Ebene quer zum Laserstrahl bzw. quer zur Erstreckung der lichtleitenden Faser erfolgen. Außerdem kann eine Verkippung um eine oder zwei Achsen erfolgen. Auch ein Fokus der lichtleitenden Faser kann eingestellt werden, um eine optimale Einkopplung des Laserstrahls in die lichtleitende Faser zu ermöglichen.

Es sei erwähnt, dass es sich bei dieser Art der Einkopplung um einen eigenständigen Erfindungsaspekt handeln kann.

Bevorzugt ist die Detektoranordnung dazu konfiguriert, ausschließlich gestreutes Licht zu detektieren. Dies hat sich in der Praxis als vorteilhaft erwiesen. Einerseits kann mittels des gestreuten Lichts eine vorteilhafte Detektion von gesuchten Zellen in der Probenflüssigkeit erfolgen. Andererseits ist durch den Verzicht auf eine Detektion transmittierenden Lichts eine kompakte Bauform möglich.

Bevorzugt weist die Richtoptik ein Fokussierelement auf, welches mittels eines Positionierelements quer zum Laserstrahl positionierbar ist und/oder innerhalb eines Tubus um eine optische Achse rotierbar ist und/oder mittels einer Gewindescheibe längs seiner optischen Achse fixierbar ist.

Durch die Verwendung eines solchen Fokussierelements können die Funktionalitäten mehrerer Komponenten zum Ausrichten und Fokussieren des Lichtstrahls in einem einzigen Element kombiniert werden. Es sei verstanden, dass dies einen eigenständigen Erfindungsaspekt darstellen kann.

Gemäß einer Weiterbildung weist die Durchflusszytometeranordnung eine Entnahmeeinheit zum Entnehmen von Probenflüssigkeit aus einem Flüssigkeitsstrom auf. Die Entnahmeeinheit kann dabei mit dem Eingangsanschluss verbunden sein. Dies ermöglicht insbesondere eine kontinuierliche Entnahme von Probenflüssigkeit aus einem Flüssigkeitsstrom, beispielsweise dem Flüssigkeitsstrom einer üblichen kommunalen Wasserversorgung.

Die Entnahmeeinheit kann insbesondere als Überstromfilter ausgebildet sein. Darunter kann insbesondere ein Element verstanden werden, durch welches der Wasserstrahl, aus welchem Probenflüssigkeit entnommen werden soll, fließt, wobei ein Filter unmittelbar an diesen Wasserstrahl angrenzt, so dass Probenflüssigkeit aus dem Wasserstrahl abgezogen werden kann. Der Wasserstrahl kann dabei insbesondere durch ein Rohr definiert sein.

Der Überstromfilter kann dabei einen Kanal mit einem Einlass und einem Auslass aufweisen. Er kann auch einen an den Kanal angrenzenden Filter aufweisen. Der Überstromfilter kann ferner einen Probenauslass aufweisen, welcher zum Abziehen von Probenflüssigkeit aus dem Kanal mit dem Eingangsanschluss verbunden ist. Dieser Probenauslass ist dabei bevorzugt mit dem an den Kanal angrenzenden Filter verbunden. Dies ermöglicht ein unmittelbares Abziehen von Probenflüssigkeit aus dem vorbeiströmenden Wasser und die Lieferung dieser Probenflüssigkeit zum Eingangsanschluss, wo die Probenflüssigkeit wie beschrieben analysiert werden kann.

Es sei verstanden, dass der hier beschriebene Überstromfilter einen eigenständigen Erfindungsaspekt darstellen kann.

Der Kanal kann insbesondere im Bereich des Filters vom Einlass zum Auslass hin verjüngt ausgeführt sein. Dadurch wird die Strömungsgeschwindigkeit kontinuierlich erhöht, was eine Blasenbildung vermeidet.

Der Kanal kann bevorzugt am Einlass einen Einlasstrichter zur Verteilung von einströmender Flüssigkeit über den Kanal aufweisen. Dadurch wird eine besonders gleichmäßige Verteilung der einströmenden Flüssigkeit über den Kanal erreicht. Auch dadurch kann eine Blasenbildung vermieden werden.

Der Auslass kann insbesondere schräg zum Kanal ausgebildet sein. Dadurch kann ein zuverlässiger Abzug von eventuell gebildeten Blasen erreicht werden. Der Auslass kann dabei insbesondere in Einbaustellung so ausgerichtet sein, dass er schräg nach oben gerichtet ist. Dadurch wird das Entfernen von Blasen schon durch deren Tendenz unterstützt, im Wasser nach oben zu steigen.

Die Vermeidung von Blasen ist besonders vorteilhaft für die Durchflusszytometrie, da Blasen die beschriebene Messung empfindlich stören würden.

Gemäß einer bevorzugten Ausführung weist die Durchflusszytometeranordnung einen Einzelprobeneinlauf auf, welcher mit dem Eingangsanschluss verbunden ist. Dies ermöglicht eine Zugabe von einzelnen Proben zum Messen, was eine Alternative zur kontinuierlichen Entnahme aus einem vorbeifließenden Wasser bzw. Wasserstrahl darstellt. Es sei verstanden, dass bei einer Durchflusszytometeranordnung beide Möglichkeiten vorgesehen sein können, d.h. es kann ein Einzelprobeneinlauf und ein Überstromfilter bzw. eine andere Entnahmeeinheit vorgesehen sein. Diese können dann alternativ verwendet werden. Hierzu können beispielsweise geeignete Ventile vorhanden sein. Es kann jedoch auch nur eine dieser beiden Möglichkeiten vorgesehen sein, oder es kann eine andere Möglichkeit zum Zuführen einer Probe vorhanden sein.

Gemäß einer bevorzugten Ausführung ist die erste Pumpe dazu ausgebildet, während eines Ansaugens des Gemisches aus dem Mischer oder dem Inkubator durch die zweite Pumpe einen Druck in dem Mischer zu erzeugen, um im Mischer und/oder im Inkubator einen konstanten Arbeitsdruck zu halten. Es kann beispielsweise auch davon gesprochen werden, dass trotz des Absaugens von Flüssigkeit durch die zweite Pumpe aus dem Mischer oder aus der Kombination aus Mischer und Inkubator der Druck konstant gehalten wird. Dies hilft zur Vermeidung von Beschädigungen am Mischer und/oder am Inkubator sowie zur Vermeidung von Blasenbildung.

Bevorzugt weist die Durchflusszytometeranordnung eine Reinigungsflüssigkeitsversorgungsvorrichtung auf, welche mit der ersten Pumpe und/oder der zweiten Pumpe verbunden ist, um Reinigungsflüssigkeit, insbesondere Reinstwasser, in die jeweilige Pumpe zu liefern. Anstelle von Reinstwasser kann beispielsweise auch eine spezielle Reinigungsflüssigkeit, beispielsweise mit Chlor, Ozon oder einer anderen oxidierenden Substanz, verwendet werden. Dadurch kann eine Reinigung von Pumpen oder anderen Komponenten erreicht werden, wobei verstanden sei, dass die jeweilige Reinigungsflüssigkeit beispielsweise auch von den Pumpen in weitere Komponenten wie beispielsweise den Mischer, den Inkubator oder die Durchflussmesszelle abgegeben werden kann.

Bevorzugt weist die Durchflusszytometeranordnung einen Entsorgungstank auf, welcher mit der ersten Pumpe und/oder der zweiten Pumpe zum Entsorgen von Flüssigkeit aus den Pumpen verbunden ist.

Dadurch kann nicht mehr benötigte Flüssigkeit zuverlässig entsorgt werden, wobei es sich dabei beispielsweise um nicht mehr benötigte Probenflüssigkeit, nicht mehr benötigten Farbstoff, nicht mehr benötigtes Gemisch oder auch um verwendete Reinigungsflüssigkeit handeln kann.

Der Entsorgungstank kann beispielsweise als Teil der Durchflusszytometeranordnung ausgebildet sein, wobei verstanden sei, dass beispielsweise auch ein direkter Anschluss an eine Ableitung wie beispielsweise eine Abwasserversorgung zum Entsorgen verwendet werden kann. Dabei wird vorteilhaft ein Aktivkohlefilter zur Beseitigung des Farbstoffs zwischengeschaltet.

Gemäß einer bevorzugten Ausführung weist die Durchflussmesszelle eine Probenkanüle zur Abgabe der Probenflüssigkeit auf, welche in einer Fassung gelagert ist. Die Fassung kann beispielsweise mittels zweier O-Ringe gelagert sein. Außerdem kann sie mittels eines Gewindes positionierbar sein.

Es sei verstanden, dass unter der hierin beschriebenen Durchflussmesszelle ein eigenständiger Erfindungsaspekt verstanden werden kann.

Die Lagerung in der Fassung hat sich als vorteilhaft herausgestellt, um einen Einsatz in unterschiedlichen und insbesondere nicht konstanten Umgebungsbedingungen zu ermöglichen. Beispielsweise wird die Stabilität gegenüber Temperaturschwankungen oder Erschütterungen erhöht. Durch das Gewinde ist eine einfache Positionierung der Probenkanüle möglich, so dass der Probenstrahl zuverlässig eingestellt werden kann, und zwar auch während der Messung. Die Fassung kann dazu beispielsweise in ihrer Umgebung gedreht werden, wodurch mittels des Gewindes auch die Position eingestellt werden kann. Mittels der O-Ringe kann eine zuverlässige Abdichtung hergestellt werden, wobei verstanden sei, dass auch eine andere Anzahl von O-Ringen verwendet werden kann.

Die Komponenten der Durchflusszytometeranordnung, insbesondere der Durchflussmesszelle, sind vorzugsweise nichtmetallisch und/oder aus Kunststoff, Glas oder Keramik ausgebildet. Dies hat sich als vorteilhaft erwiesen, da metallische Bestandteile, welche in Kontakt mit Probenflüssigkeit kommen, aufgrund fehlender Biokompatibilität die Funktionalität beeinträchtigen können.

Bevorzugt weist die Durchflusszytometeranordnung eine Steuerungseinrichtung auf, welche dazu konfiguriert ist, die Pumpen zu steuern. Dadurch kann ein automatisierter Betrieb der Pumpen erreicht werden. Beispielsweise kann eine solche Steuerungseinrichtung als üblicher Computer, als Mikrocontroller, Mikroprozessor, speicherprogrammierbare Steuerung (SPSS), anwendungsspezifischer integrierter Schaltkreis (ASIC) oder als andere programmierbare oder festverdrahtete Vorrichtung ausgeführt sein. Insbesondere kann sie Prozessormittel und Speichermittel enthalten, wobei in den Speichermitteln Programmcode gespeichert ist, bei dessen Ausführung sich die Prozessormittel in definierter Weise verhalten.

Es sei verstanden, dass die Steuerungseinrichtung auch andere Funktionen als die Steuerung der Pumpen wahrnehmen kann, beispielsweise eine Überwachung von Parametern oder eine Steuerung von Ventilen oder anderen Komponenten.

Die Steuerungseinrichtung ist bevorzugt dazu konfiguriert, die erste Pumpe und die zweite Pumpe derart zu steuern, dass sie gleichzeitig vorgegebene Volumina an Probenflüssigkeit und Farbstoff in den Mischer pumpen. Dadurch kann eine definierte Befüllung des Mischers erreicht werden, so dass genau definierte Mischungsverhältnisse eingestellt werden können.

Die Steuerungseinrichtung kann bevorzugt dazu konfiguriert sein, die erste Pumpe und die zweite Pumpe derart zu steuern, dass die zweite Pumpe Gemisch aus dem Mischer oder Inkubator ansaugt und durch synchronen Betrieb der ersten Pumpe und der zweiten Pumpe ein Betriebsdruck im Mischer und/oder Inkubator auf einem vorgegebenen Druck gehalten wird. Wie bereits weiter oben beschrieben, können dadurch Beschädigungen des Mischers und/oder des Inkubators sowie Blasenbildung vermieden werden, da Druckschwankungen verhindert werden.

Es sei verstanden, dass beim Halten von vorgegebenen Drücken grundsätzlich gewisse Toleranzen technisch unvermeidbar sind.

Bevorzugt ist die Steuerungseinrichtung dazu konfiguriert, die zweite Pumpe derart zu steuern, dass die zweite Pumpe Gemisch aus dem Mischer oder Inkubator ansaugt. Dadurch kann ein Gemisch in die zweite Pumpe gelangen, insbesondere um anschließend als Probenstrahl verwendet zu werden.

Bevorzugt ist die Steuerungseinrichtung dazu konfiguriert, das Pumpen von Probenflüssigkeit und Farbstoff in den Mischer zuerst zu veranlassen, anschließend eine vorbestimmte Inkubationszeit zu warten, und anschließend das Ansaugen des Gemischs zu veranlassen. Das Ansaugen kann insbesondere durch die zweite Pumpe erfolgen. Durch das Abwarten der Inkubationszeit wird in vorteilhafter Weise ermöglicht oder verbessert, dass sich Farbstoffe an zu detektierende Partikel binden.

Bevorzugt ist die Steuerungseinrichtung dazu konfiguriert, die erste Pumpe so zu steuern, dass sie Hüllflüssigkeit in die Durchflussmesszelle pumpt, und gleichzeitig die zweite Pumpe so zu steuern, dass sie aus dem Mischer oder Inkubator angesaugtes Gemisch in die Durchflussmesszelle pumpt. Dadurch kann ein üblicher Betrieb einer Durchflussmesszelle mit Probenstrahl und Hüllstrahl mit nur zwei Pumpen realisiert werden.

Bevorzugt weist die Durchflusszytometeranordnung ferner eine Auswerteeinrichtung auf, welche dazu konfiguriert ist, mittels der Durchflusszytometeranordnung gewonnene Messerergebnisse auszuwerten. Dadurch kann eine automatisierte Auswertung der Messergebnisse erreicht werden, was beispielsweise eine weitere Automatisierung der Überwachung einer Trinkwasserversorgung ermöglicht.

Bezüglich der Ausgestaltung der Auswerteeinrichtung gilt das weiter oben für die Steuerungseinrichtung erwähnte entsprechend. Es sei verstanden, dass Steuerungseinrichtung und Auswerteeinrichtung auch als eine Einheit bzw. eine Komponente ausgeführt sein können.

Die Auswerteeinrichtung kann insbesondere dazu konfiguriert sein, zweidimensionale Plots zu erzeugen. Auf einer ersten Achse eines solchen Plots ist dabei ein erster Kanal aufgetragen und auf einer zweiten Achse ist ein zweiter Kanal aufgetragen. In dem Plot werden dabei an jeweiligen Punkten eine Anzahl von gemessenen Werten aufgetragen. Dies kann beispielsweise bedeuten, dass jeder Kanal einem Detektor zugeordnet wird und die Intensität somit den zweidimensionalen Plot definiert. Bei jeder Kombination der beiden Intensitäten wird die jeweils gemessene Anzahl derartiger Intensitätskombinationen in einem bestimmten Zeitfenster angetragen. Daraus ergeben sich charakteristische Muster, welche Aufschluss über die Trinkwasserqualität liefern. Insbesondere können bestimmte unerwünschte Arten von Zellen wie bestimmte Bakterien in bestimmten Bereichen derartiger Plots gefunden werden, so dass eine Identifizierung leicht möglich ist. Dadurch kann sehr schnell auf gewisse Ereignisse reagiert werden.

Die Auswerteeinrichtung kann insbesondere dazu konfiguriert sein, in den Plots Bereiche zu definieren, wobei bei Überschreiten einer Anzahl an gemessenen Werten innerhalb eines Bereichs eine vordefinierte Aktion ausgelöst wird. Bei einer solchen vordefinierten Aktion kann es sich beispielsweise um einen Alarm handeln. Es kann sich auch um eine andere Aktion wie beispielsweise die Abschaltung der Trinkwasserversorgung oder das Zuschalten von bestimmten Desinfektionsmaßnahmen handeln.

Es sei verstanden, dass durch die erfindungsgemäße Durchflusszytometeranordnung ein System geschaffen wird, welches eine automatisierte, zuverlässige und kontinuierliche sowie schnelle Überwachung der Trinkwasserqualität ermöglicht. Eine Messung, wie sie mit der hierin beschriebenen Durchflusszytometeranordnung erfolgen kann, kann beispielsweise in der Größenordnung von 15 Minuten bis 25 Minuten, insbesondere 20 Minuten, durchgeführt werden. Dies ermöglicht eine nahezu instantane Erkennung von Ereignissen, welche die Trinkwasserqualität negativ beeinflussen, und ermöglicht somit eine sofortige Reaktion und ein Einleiten von geeigneten Gegenmaßnahmen. Die Sicherheit der Trinkwasserversorgung wird damit deutlich erhöht.

Es sei des Weiteren verstanden, dass mittels der Durchflusszytometrie, insbesondere wie hierin beschrieben, alle oder zumindest nahezu alle in der Probenflüssigkeit vorhandenen Zellen detektiert werden können. Dies stellt einen signifikanten Vorteil gegenüber der bekannten AMK-Methode dar. Durch die signifikante Reduzierung der Messdauer von ca. 72 Stunden auf wenige Minuten stehen dem Anwender nun präzise, realistische Resultate über die mikrobielle Flora im Wasser online zur Verfügung. Bis dahin war dies nur für chemische und physikalische Parameter möglich.

Durchflusszytometrie kann insbesondere eingesetzt werden, um in Wasserproben die Gesamtzellzahl (GZZ), High Nucleic Acid (HNA), Low Nucleic Acid (LNA) und lebende/tote Zellen zu bestimmen. Es ist auch möglich, mittels spezifisch andockender Farbstoffe spezifische Zellen zu erkennen.

Die erfindungsgemäße Durchflusszytometeranordnung ist insbesondere am Ort der Probenentnahme, d.h. außerhalb eines Labors, verwendbar und rund um die Uhr einsatzbereit. Des Weiteren kann damit eine robuste und präzise durchflusszytometrische Detektion erfolgen. Es kann eine automatisierte Probenvorbereitung wie Probenentnahme, Anfärben und Inkubieren vorgesehen sein, es kann eine automatisierte Auswertung der Messresultate vorgesehen sein, es kann eine Alarmierung bei Ereignissen vorgesehen sein, es kann eine Datenarchivierung, beispielsweise in einer Datenbank, vorgesehen sein, es können Schnittstellen zur Prozesssteuerung vorgesehen sein, und es kann eine Überwachung der Fluide wie beispielsweise Reinigungslösung, Reinstwasser, Hüllflüssigkeit oder Farbstoff vorgesehen sein. Die erfindungsgemäße Durchflusszytometeranordnung ist typischerweise für einen ständigen Betrieb, also beispielsweise an 24 Stunden pro Tag und an 7 Tagen die Woche, ausgelegt, und sie kann immun gegen Temperaturschwankungen und Bedingungen in industriellen Umgebungen sein. Außerdem kann ein Fernzugriff, beispielsweise über Ethernet oder andere Netzwerktechnologien, zur Verfügung gestellt werden.

Es wird ein zuverlässiges Messsystem für den Vor-Ort-Einsatz vorgesehen, das den allgemeinen und hygienischen mikrobiellen Zustand des Wassers und die Bakterienaktivität in kurzzeitlichen Abständen erfasst und zur Verfügung stellt.

Insbesondere kann die beschrieben Durchflusszytometeranordnung zum Einsatz außerhalb von Laboren, etwa bei Wassergewinnung, Wasseraufbereitung, Wasserdistribution oder in industriellen Anlagen geeignet sein und daher gegen an diesen Orten herrschende physikalische Gegebenheiten wie Temperatur, Temperaturschwankungen, schwankende Luftfeuchtigkeit, Staub, Partikel und Erschütterungen immun sein. Es können mittels Durchflusszytometrie Partikel und Bakterien auch außerhalb von Laboratorien präzise detektiert werden.

Durchflusszytometrie basiert grundsätzlich auf der Einfärbung von lebenden und toten Zellen mittels DNA-Farbstoffen, die sich an die intakte oder beschädigte DNA binden.

Mithilfe von SYBR Green-spezifischer Grünfluoreszenz können mikrobiologische, intakte Zellen von DNA-freien, anorganischen Partikeln unterschieden werden. Beispielsweise kann Propidiumiodid (PI) die perforierte bzw. beschädigte Zellmembran von toten Zellen, jedoch nicht die intakte Membran von lebenden Zellen durchdringen. Diese Eigenschaft kann in der Durchflusszytometrie, beispielsweise bei der hierin beschriebenen Anordnung, zur Lebend-/Tot-Unterscheidung von Zellen angewendet werden.

Die Mikroorganismen und Partikel werden nach dem Anfärben und Inkubieren typischerweise einzeln durch eine Glaskapillare geschleust und mit einem fokussierten Laserstrahl beleuchtet, um den DNA-Farbstoff anzuregen. Trifft der Laser auf eine Zelle oder ein Partikel, wird das gestreute Licht im Streulichtkanal erfasst und das emittierte Licht der Zellen über die Fluoreszenzkanäle detektiert. Mittels der erfindungsgemäßen Durchflusszytometeranordnung kann auch erreicht werden, dass sämtliche Prozessschritte für einen komplett automatisierten Online-Betrieb in ein kompaktes, industrietaugliches Messgerät integriert werden können. Als komplett automatisiert soll insbesondere im Zusammenhang mit der vorliegenden Beschreibung verstanden werden, dass das Gerät über einen Zeitraum von mindestens zwei Wochen ohne personelle Intervention störungsfrei funktioniert. Darüber hinaus soll auch das Problem gelöst werden, dass die einzelnen Prozessschritte für einen Fail-Safe-Betrieb ausgelegt werden sollen und hierfür mögliche Störeinflüsse, die zu einem Ausfall führen könnten, abgefangen bzw. kompensiert werden.

Die Durchflusszytometeranordnung kann beispielsweise in einem kompakten Gehäuse eine oder mehrere Spritzenpumpen bzw. Spritzenpumpeneinheiten, wenigstens ein Lasermodul, Mittel zur Datenerfassung (DAQ), ein Motor Control Board, eine neuartige, kompakte optische Detektionseinheit, wenigstens eine Mischeinheit zur Probenauf- und -vorbereitung, wenigstens einen Inkubator, eine Instrument Control (Steuerung), wenigstens einen Temperaturregler für die Messreaktion und/oder Proben, Anwendungsschnittstellen zur Durchführung und Steuerung der Messroutinen, Steuerung der Vorrichtungskomponenten, Erfassung sowie Auswertung der Probenparameter, Weitergabe der Messerergebnisse und Daten sowie gegebenenfalls der Ausgabe von Alarmmeldung enthalten. Es sei verstanden, dass auch eine beliebige Unterkombination der eben genannten Komponenten enthalten sein kann. Um einen laborfernen Einsatz außerhalb standardisierter Umweltbedingungen zu ermöglichen, können sämtliche Komponenten in einem kompakten, industrietauglichen Instrumentengehäuse untergebracht werden, wobei die Einzelkomponenten ebenfalls eine kompakte und bauraumoptimierte Größe aufweisen. Durch geeignete Zu-/Abführung von Luft und/oder Wärme sowie Komponentenkühlung bzw. Komponentenheizung werden die Bedingungen im Inneren des Gehäuses eingestellt, um eine möglichst gleichmäßige Messumgebung zur Verfügung zu stellen, die von den Bedingungen am Aufstellort des Geräts unabhängig ist.

Die Erfindung stellt beispielsweise auch ein Verfahren zur Messung mikrobiologischer und/oder physikalischer Probenparameter und/oder zur anschließenden Datenauswertung zur Verfügung.

Jedes Wasser variiert in seiner mikrobiologischen Struktur. Es können nach dem Erfassen der einzelnen Events mittels durchflusszytometrischer Detektion die Signale zweier Messkanäle anhand ihrer Signalstärke dargestellt und je nach Kombination der Messkanäle entsprechende Fluoreszenz- und Streulicht-Plots erstellt werden. Es kann dabei vorgesehen sein, diese Plots mittels Gates (Bereiche), die in die Plots eingegrenzt werden, auszuwerten. Gates sollen in den jeweiligen Plots entsprechend definiert und ihre Grenzwerte festgelegt werden.

Das erfindungsgemäße Verfahren kann beispielsweise zur Auswertung via Gates Folgendes vorsehen:
- Erfassung/Darstellung der Anzahl der Events in allen definierten Gates,
- Unterscheiden von LNA- und HNA-Bakterien,
- Unterscheiden von aktiven und beschädigten Zellen.

Durch wertmäßige Festlegung einzelner Regionen in den Gates und der Kombination unterschiedlicher Gates in einzelnen oder über mehrere Plots können in einer bevorzugten Weiterbildung Warnungs- und Alarmbedingungen definiert werden, wobei insbesondere dann, wenn im zu messenden Wasser bzw. Fluid individuelle, vordefinierte Bedingungen erreicht werden, automatisch eine Warnung oder ein Alarm ausgelöst werden.

Solche Ereignisse (zum Beispiel Warnungen und/oder Alarme) können bevorzugt wie folgt ausgegeben und weitergeleitet werden:
- Senden eines Messprotokolls via E-Mail,
- akustischer/optischer Alarm (zum Beispiel Lampe am Gerät),
- Ausgabe von analogen und digitalen Signalen (zum Beispiel an eine SPS),
- via Ethernet und/oder kundenseitiges Leitsystem.

Ereignis-Definitionen können beispielsweise an den jeweiligen Plot bzw. an das zu messende Wasser adaptiert werden, denn jedes Wasser weist unterschiedliche Wolkenbilder bzw. Fingerprints auf. Bei derartigen Wolkenbildern bzw. Fingerprints kann es sich insbesondere um die Darstellungen der genannten Plots handeln.

Ein Messprinzip kann vorsehen, dass durch Differenzmessungen zweier und mehrerer Messungen die jeweiligen Messdaten zueinander in Beziehung gebracht werden. So ergeben beispielsweise Messungen gleicher Wasserproben im Zeitlauf, beispielsweise alle 30 Minuten, bei konstanten Bedingungen nur geringfügige Änderungen. Tritt beispielsweise ein Ereignis ein - etwa die Infiltration von Regenwasser, Kontamination mit Jauche oder eine funktionale Störung im Aufbereitungsprozess - verändern sich die darauffolgenden Fluoreszenz- und Streulicht-Plots in kurzer Zeit. Auf mikrobiologische Veränderungen kann so unverzüglich reagiert werden.

Warnungs- und Alarmkriterien können insbesondere bedarfsweise konfiguriert und ausgegeben werden.

Ein Verfahren kann insbesondere einzeln oder in Kombination die Erfassung und/oder Auswertung der Gesamtzahl aller Events (detektierte Partikel und/oder Zellen), Gesamtzahl der Zellen (aktiv und beschädigt) oder Totalerfassung aller Partikel (Partikelmesskanal) ermöglichen.

Durch Definition von Gates kann zudem in einer vorteilhaften Weiterbildung die Anzahl der Events (detektierte Partikel und/oder Zellen) in allen definierten Gates, ein Unterscheiden von aktiven und beschädigten Zellen sowie ein Unterscheiden von LNA- und HNA-Bakterien ermöglicht werden.

Die grafische Darstellung von Messresultaten erfolgt bevorzugt als 2D-Plot bzw. die Plots sind bevorzugt 2D-Plots. Sie können jedoch auch 3D-Plots, Histogramm-Plots, History-Plots und/oder Density-Plots sein.

Günstigerweise können Daten als PDF-Messprotokoll, CSV-Messprotokoll wie Ethernet und/oder via Fernzugriff ausgegeben werden.

Es ist auch möglich, eine Unterscheidung in intakte und beschädigte Zellen vorzunehmen. Dies kann beispielsweise sinnvoll sein, um eine Überprüfung von Desinfektionsmaßnahmen, beispielsweise mittels Ozonierung oder Chlorierung, durchzuführen. Hierfür kann ein Nachweis von lebensfähigen Zellen durchgeführt werden. Oxidantien verursachen bei Bakterien eine schwere Schädigung der Zellwände. Mit Propidiumiodid lassen sich selektiv nur die membrangeschädigten Zellen anfärben und so von intakten Bakterien unterscheiden. Die durchflusszytometrische Detektion, kombiniert mit Färbemethoden, ermöglicht auch Aussagen über die Aktivität bzw. Lebensfähigkeit von Mikroorganismen im Trinkwasser. Die hierin beschriebene Durchflusszytometeranordnung erlaubt es, die Kinetik der Desinfektionen zu untersuchen und zu verstehen. Insbesondere erhalten damit Wasserversorgungen eine kompakte und robuste Vorrichtung sowie ein zugehöriges Verfahren, womit Desinfektionsmaßnahmen evaluiert und direkt vor Ort überprüft werden können. Es sei verstanden, dass dies auch einen eigenständigen Erfindungsaspekt darstellen kann.

Die Erfindung betrifft auch ein Verfahren und eine Vorrichtung zum synchronen Betreiben zweier oder mehrerer elektrisch gekoppelter und motorgetriebener Spritzeneinheiten. Diese können insbesondere im Rahmen der hierin beschriebenen Durchflusszytometeranordnung verwendet werden, können jedoch auch als eigenständige Erfindung aufgefasst werden.

Bei einer solchen Spritzeneinheit kann ein synchroner Betrieb von mindestens zwei elektrisch gekoppelten, motorbetriebenen Spritzeneinheiten vorgesehen sein, um - insbesondere in der hierin beschriebenen Durchflusszytometeranordnung - Fluide unter anderem präzise zu mischen, beispielsweise in einer speziellen, insbesondere in der hierin beschriebenen Durchflusszytometeranordnung optional vorgesehenen Misch- und Reaktoreinheit, zu fokussieren und zu transportieren. Die Verwendung der erfindungsgemäßen Spritzeneinheit bleibt dabei nicht auf Durchflusszytometer beschränkt.

Bekannte Spritzeneinheiten werden für vielfältige Aufgaben eingesetzt, zum Beispiel um Fluid kontinuierlich über die Zeit auszustoßen, beispielsweise zur Dosierung. Dabei kann beispielsweise über eine Kontrollapplikation die Spritzeneinheit parametrisiert und der Prozess gestartet werden. Relevante Parameter können dabei beispielsweise ein Volumen, das aufzuziehen ist bzw. ausgestoßen werden soll, und eine Geschwindigkeit (Volumen pro Zeiteinheit), in der dies geschehen soll, sein. Zusätzlich kann eine Spritzeneinheit mit einem zusätzlichen motorbetriebenen Rotationsventil ausgestattet sein, um Fluide von unterschiedlichen Quellen aufzuziehen bzw. um die Ausgabe an unterschiedliche Zielleitungen zu ermöglichen. Um ruckartige Bewegungsabläufe beim Start bzw. beim Ende zu vermeiden, ist es üblich, sogenannte Rampen einzusetzen, welche beispielsweise einer Beschleunigung beim Starten entsprechen, bis die gewünschte maximale Geschwindigkeit erreicht ist.

Mit der hierin beschriebenen Ausführung ist es neben dem Einzelbetrieb einer solchen Spritzeneinheit möglich, zwei oder mehr Pumpen- bzw. Spritzeneinheiten synchron zu betreiben. Die Vorteile eines solchen parallel synchronen Betriebs sind zum einen die Möglichkeit, zwei oder mehr Fluide sehr präzise in einem gegebenen Verhältnis zu mischen. Hierbei können bevorzugt Mischkomponenten (zum Beispiel Butterfly-Mischer) verwendet werden, welche konstante Geschwindigkeiten der zu mischenden Fluide benötigen, um eine hohe Durchmischung zu gewährleisten. Ein weiterer Vorteil des synchronen Betriebs ist die Möglichkeit, Fluide von einer Spritzeneinheit in eine andere zu transportieren. Hierbei erweist es sich als vorteilhaft, wenn eine (Ausgangs-)Spritzeneinheit das zu transportierende Fluid drückt, während die (Ziel-)Spritzeneinheit aufzieht. Hierbei ist die Präzision der Synchronisierung von entscheidender Bedeutung, da durch Abweichungen hohe Drücke entstehen können. Zusätzlich können durch einen synchronen Ausstoß zweier Flüssigkeiten hydrodynamische Effekte erzielt werden, wie sie zum Beispiel in der Durchflusszytometrie benötigt werden.

Die Erfindung betrifft auch eine Detektionseinheit, welche insbesondere in der hierin beschriebenen Durchflusszytometeranordnung verwendet werden kann, welche jedoch auch als eigenständige Erfindung aufgefasst werden kann.

Die Detektionseinheit kann insbesondere einen Optik-Monoblock umfassen oder als solcher ausgebildet sein. Insbesondere kann vorgesehen sein, dass für eine durchflusszytometrische Detektion notwendige Module und Komponenten, insbesondere Durchflusszelle, optische Filter, Detektoren, Transimpedanzverstärker, Signalerfassung- und -verarbeitung, Laser und Lichteinkoppeloptik, insgesamt oder teilweise in einer neuartigen, symmetrischen Anordnung zu einer bevorzugt aus einem Vollmaterial gearbeiteten, insbesondere gefrästen Detektionseinheit, die bevorzugt als Monoblock ausgebildet ist, vereint werden. Die Detektionseinheit wird so nicht nur kompakt und weitgehend staubdicht, sondern auch mechanisch hochstabil.

Detektionseinheiten und deren Komponenten können ortsfest in einem Gehäuse angeordnet sein, das einteilig oder einstückig aus einem Vollmaterial gefertigt ist. Es können auch Detektionseinheiten verwendet werden, deren Gehäuse aus zwei oder mehr Teilen besteht. Insbesondere kann vorgesehen sein, dass sämtliche Bestandteile der Detektionseinheit im Gehäuse untergebracht sind. Die erfindungsgemäße Ausführung ist hierauf jedoch nicht beschränkt, sondern umfasst auch andere Ausführungsvarianten, bei denen einzelne Komponenten außerhalb des Monoblocks bzw. Gehäuses angeordnet oder vorgesehen sind.

Im Vollmaterial können gemäß einer bevorzugten Ausführungsform die mechanischen Austragungen im Grundkörper nur dort vollzogen werden, wo sie notwendig sind, damit die mechanische Steifigkeit so hoch wie möglich bleibt. Je höher die mechanische Stabilität, desto präziser kann das Gerät arbeiten.

Eine Monoblock-Konstruktion und die damit erlangte höhere Stabilität und Kompaktheit lassen auch lokale Anwendungen in industriellen Umgebungen zu. Die größere Immunität gegenüber den physikalischen Gegebenheiten Temperatur, Luftfeuchtigkeit, Staub und Erschütterungen erfüllt die erhöhten Anforderungen der einzelnen industriellen Szenarien.

Gemäß einer Ausführung wird ein symmetrisches Design zweier gegenüberliegender Objektive für jeweils zwei optische Messkanäle realisiert. Dieses benötigt beispielsweise lediglich noch je einen Strahlteiler und zwei Farbfilter.

Durch Reduktion der Strahlteiler und Spiegel kann die optische Effizienz gesteigert werden.

Der Optik-Monoblock kann sämtliche für eine durchflusszytometrische Detektion notwendige Komponenten und Module enthalten, beispielsweise Lichtquelle, Lasermodul, Durchflussmesszelle, Lichteinkopplung, Strahlteiler, Filter, optische Detektoren mit Transimpedanzverstärkern, Datenerfassung und -verarbeitung (DAQ). Ein FCM-Gerät ist bevorzugt in sich vollständig abgeschlossen.

Die Erfindung betrifft auch eine Doppelschlitz-Streulichtblende. Diese kann insbesondere in der hierin beschriebenen Durchflusszytometeranordnung verwendet werden. Hierauf ist sie jedoch nicht eingeschränkt, sie kann vielmehr auch als eigenständiger Erfindungsaspekt aufgefasst werden.

In der Durchflusszytometrie ist außer den Fluoreszenz-Farbmesskanälen typischerweise auch das vorwärts und seitlich gestreute Licht von Interesse. Das vorwärts gestreute Licht gibt Aufschluss über die Größe von Bakterien und Partikeln, das seitliche hingegen über ihre Struktur und Form.

Heute werden in durchflusszytometrischen Anordnungen typischerweise vor die optischen Detektoren Loch-Schlitz- oder Stegblenden platziert, welche entweder horizontal oder vertikal positioniert sein können, um das direkte, vorwärts gestreute oder das indirekte, seitlich gestreute Licht abzudunkeln.

Diese Art von Lichtunterdrückung bedeutet immer einen Kompromiss zwischen zu hohem Signal-Offset und zu starker Unterdrückung des Nutzsignals. Aufgabe der erfindungsgemäßen Doppelschlitz-Streulichtblende ist es dabei beispielsweise, eine optimale Signal-Offset-Unterdrückung zur Verfügung zu stellen, ohne das Nutzsignal zu beeinträchtigen. Es ist bevorzugt eine Integration der Blende in Kombination mit einem optischen Filter in bestehende Detektionseinheiten, bevorzugt einen erfindungsgemäßen Optik-Monoblock, vorgesehen.

Dies kann dadurch erreicht werden, dass es eine Vorrichtung ermöglicht, in der Durchflusszytometrie das seitlich und vorwärts gestreute Licht so auf den Detektor treffen zu lassen, dass ein statischer Signal-Offset unterdrückt wird und das gestreute Licht, verursacht durch Partikel und Bakterien, ohne Beeinträchtigung der Signalqualität auf den Detektor trifft

Die erfindungsgemäße Doppelschlitzblende kann mittels einer integrierten, vertikalen Führung exakt in die Höhe des Lichtstrahls justiert werden, um ein optisches Nutzsignal zu erhalten und Störungen durch Streulicht auszuschließen.

Vorteilhaft ist mit der Doppelschlitz-Streulichtblende nahezu die gesamte Bandbreite der optischen Messsignale nutzbar, denn direkt in die optischen Detektoren reflektiertes Licht wird vermieden.

Eine Detektionseinheit bzw. Detektoranordnung wie hierin beschrieben umfasst vorteilhafterweise auch als Lichtquelle einen Laser mit integrierter Faserkopplung. Die Verwendung ist jedoch nicht auf die Detektionseinheit bzw. Detektoranordnung wie hierin beschrieben beschränkt, sondern kann auch als eigenständiger Erfindungsaspekt angesehen werden.

Um Zellen mit einen DNA- oder RNA-Kern von Partikeln zu unterscheiden, werden in der Durchflusszytometrie typischerweise die mit Fluoreszenzfarbstoff gefärbten Zellen durch Laserlicht mit eng definierten Wellenlängen angeregt. Diese monochromatische Lichtfrequenz bzw. die Wellenlänge muss äußerst stabil sein, um Messfehler in der Signalerfassung zu vermeiden.

Fasereinkopplungen der Lichtquelle erfolgen durch vom Laser komplett abgesetzte oder im Lasermodul intern entkoppelte Justiereinheiten, die meistens laterale Positionierungen (x- und y-Richtung) sowie Positionierungen in z-Richtung und Verkippung ermöglichen. Nachteilig bei Anordnung der Laserlichtquelle in einem Lichtleiter (meistens Single Mode-Faser) ist, dass diese Art der Kopplung empfindlich auf Erschütterung, Vibrationen und Temperaturschwankungen reagiert. Ihr Einsatz in Labordurchflusszytometern funktioniert jedoch dank guter standardisierter Bedingungen im Laborumfeld meistens einwandfrei.

Da eine durchflusszytometrische Detektion außerhalb von Labors und insbesondere unter nicht standardisierten Bedingungen realisiert werden soll, jedoch mit einer Durchflusszytometern im Labor entsprechenden Messgenauigkeit, soll ein Lasersystem zur Verfügung gestellt werden, das diese Anforderungen erfüllt. Ein weiteres Kriterium ist die Verlängerung der Lebensdauer der Laserlichtquelle im 24/7-Betrieb. Nachteilig an üblichen Festkörperlasern ist, dass deren Lebensdauer sich beim 24/7-Betrieb bzw. beim durchgehenden oder kontinuierlichen Betrieb auf etwa ein Jahr beschränkt. Um die Einsatzdauer auf mehrere Jahre sicherzustellen, wird bevorzugt ein Halbleiterlaser (Diode) verwendet.

Da solche Diodenlaser die anwendungsbezogene Lichtstrahlqualität lediglich in Kombination mit einer abgestimmten Single Mode-Faser erbringen, wird als Lösung ein fasergekoppeltes Diodenmodul zur Verfügung gestellt.

Dieses fasergekoppelte Diodenmodul kann auch als Diodenlasermodul mit integrierter Faserkopplung bezeichnet werden. Ein Laserdiodensupport ist dabei bevorzugt so konzipiert, dass er nicht nur Laserdiode und Kollimationslinse beinhaltet, sondern auch eine Fokussierlinse und die Faserpositionierung. Die in den Laserdiodensupport integrierte Faserkopplungseinheit beinhaltet die laterale Positionierung (x-, y-Richtung), die Fokussierung und eine Winkelverstellung in beiden Achsen.

Von der Rückseite der Module bzw. Vorrichtung her zugängliche Feinstellschrauben sind vorzugsweise für die Winkelverstellung und Fokussierung zuständig. Die oben und seitlich angebrachten Feinstellschrauben dienen der lateralen Positionierung.

Nach abgeschlossener Positionierung des Lichtleiters in der Detektionseinheit wird mittels Fixierschrauben, die ebenfalls von der Rückseite zugänglich sind, die laterale Justiereinheit mit dem Laserdiodensupport verschraubt.

Indem bevorzugt alle Funktionen, insbesondere Laserdiode, Kollimations- und Fokussierlinse sowie Faserkopplungseinheit, in einem Körper vereint sind, können Störeinflüsse wie Vibration oder mechanische Verschiebungen durch thermische Einflüsse abgefangen werden. Eine ausreichende Langzeitstabilität, insbesondere für durchflusszytometrische Anwendungen, kann somit erreicht werden.

Ein weiterer Vorteil ist die höhere Kompaktheit der Anordnung, die höhere mechanische Stabilität sowie die weitgehende Unempfindlichkeit bezüglich Temperaturschwankungen und Vibrationen.

Die hierin beschriebene Detektionseinheit bzw. Detektoranordnung umfasst vorteilhafterweise auch eine Durchflussmesszelleneinheit. Die Verwendung ist jedoch nicht auf eine Detektionseinheit bzw. Detektoranordnung wie hierin beschrieben beschränkt, sondern kann auch als eigenständiger Erfindungsaspekt betrachtet werden.

Eine Durchflussmesszelle bzw. Durchflussmessküvette ist eine zentrale, fluidisch-optische Komponente einer durchflusszytometrischen Anordnung.

Die Durchflussmesszelle beinhaltet typischerweise die Zuführung der Proben- und Hüllstromflüssigkeit, eine Quarzglasküvette sowie die mechanische Zusammenführung dieser Komponenten und Funktionen.

Die Durchflussmesszelle stellt sicher, dass die Zellen einer Probenflüssigkeit einzeln und immer im Zentrum (im optischen Fokus) durchgeschleust werden. Dies geschieht durch eine sogenannte hydrodynamische Fokussierung. Die geometrischen Abmessungen und Verhältnisse der Proben- und Hüllstromzuführungen beeinflussen den Probenstrahldurchmesser, die Hüllstrommenge und die Stabilität des Probenstrahls. Die Probenflüssigkeit wird in eine schneller fließende Trägerflüssigkeit injiziert und durch eine Verengung im Zuführungskanal innerhalb der Durchflusskammer geführt. Diese Verengung erhöht die Durchflussgeschwindigkeiten proportional zur Querschnittsänderung des Kanals. So ändert sich auch das Querschnittsverhältnis zwischen Probenflüssigkeit und Trägerflüssigkeit in Abhängigkeit zur relativen Durchflussrate.

Die Durchflussraten werden bevorzugt so eingestellt, dass die Reduktion des Probenquerschnitts die Zellen dazu zwingt, sich innerhalb der Flüssigkeit einzureihen. Zusätzlich erhöht sich durch die Geschwindigkeitsänderung auch der Abstand zwischen den einzelnen Zellen.

Der große Vorteil der hydrodynamischen Fokussierung ist, dass die Durchflussraten sehr gut eingestellt und die Probenflüssigkeit sehr stabil geführt werden können.

Insbesondere kann die Durchflussmesszelle in durchflusszytometrischen Anordnungen bzw. Durchflusszytometeranordnungen zum präzisen Erfassen von einzelnen Zellen und Partikeln in wässrigen Lösungen eingesetzt werden.

Dabei kann es insbesondere vorteilhaft sein, einen stabilen, pulsationsfreien Probenstrahl in der Durchflussküvette im Zentrum des Messkanals zu erhalten. Hierzu ist neben einer sehr präzisen, volumenstromgeregelten Flüssigkeitszuführung die vertikale Position der Probenzuführungskanüle entscheidend.

Eine optimale Position der Probenkanüle soll nicht nur bei der Montage der Einheit vorgenommen werden können, sondern sie soll auch während des Betriebs verstellt werden können. Eine solche Durchflussmesszelleneinheit soll auch außerhalb von Labors präzise und stabil die adäquaten Messresultate liefern. Deshalb ist das Messzellendesign bevorzugt so gewählt, dass die variierenden Ausdehnungskoeffizienten der unterschiedlichen Materialien - insbesondere Quarzglas, Aluminium und Kunststoff - keine negativen Auswirkungen auf die laterale Position des Küvettenkanals haben.

Dies kann insbesondere dadurch erreicht werden, dass die Probenzuführung während einer Kalibrationsmessung vertikal positioniert wird, indem eine Nadel zur Aufgabe einer Probe mit einer lateral positionierten, geführten Probennadelfassung aus zwei O-Ringen versehen ist. Außer der Positionierung und Führung der Nadelfassung dienen die zwei an den Enden angebrachten O-Ringe als mechanische Bremse, um nach erfolgter Justierung ein zum Beispiel durch Vibrationen verursachtes, eigenständiges Dejustieren zu verhindern.

Die Probennadelfassung kann insbesondere mit einem außenseitigen Feingewinde versehen werden. Dessen Steigung ist korreliert mit einer erreichbaren vertikalen Positionierauflösung. Das Feingewinde kann in einer Ausführungsform beispielsweise eine Steigung von 0,25 mm aufweisen, um eine vertikale Positionierauflösung zu erreichen, die bevorzugt kleiner als 1 µm ist.

Bevorzugt weist die Durchflussmesszelleneinheit die Materialien PEEK, Aluminium und Quarzglas auf oder ist aus diesen gefertigt. Die Materialien können insbesondere einen nicht korrelierenden thermischen Koeffizienten aufweisen. Um dies zu kompensieren, ist der Gegenhalter der Messküvette bevorzugt federnd, insbesondere mittels Tellerfedern, angebracht. Um die laterale Stabilität des Gegenhalters sicherzustellen, ist in seinem Umfang bevorzugt wenigstens ein O-Ring montiert. Es können beispielsweise auch zwei O-Ringe montiert werden. So kann erreicht werden, dass die Längenvarianz der unterschiedlichen Materialien über einen Temperaturbereich von insbesondere ca. 60 °C bis 70 °C kompensiert wird.

Die Hüllstromflüssigkeit, die an einer Stelle über einen Kanal in den zirkularen Verteilring eingeleitet wird, soll bevorzugt Probenkanüle und Probenstrahl homogen umströmen. Daher wird die Flüssigkeit bevorzugt durch eine Lochblende geradegerückt.

Die Erfindung umfasst auch ein Verfahren, welches beispielsweise mittels der erfindungsgemäßen Durchflusszytometeranordnung durchführbar ist, zur Auswertung von flusszytometrischen Streukanalmessdaten, die bevorzugt zur Bestimmung der Trübung von Flüssigkeiten dient. Das Verfahren weist dabei eine eigenständige erfinderische Bedeutung auf und ist nicht auf die Verwendung mit der hierin beschriebenen Durchflusszytometeranordnung beschränkt.

Die bei der Durchflusszytometrie anfallenden Messdaten werden zusätzlich für die Bestimmung der Trübung verwendet.

Die Durchflusszytometrie (DFZ) soll bevorzugt unter anderem im Sektor der Wasserqualitätsbestimmung vermehrt veraltete Methoden ablösen. Diese vornehmlich aus der Medizin bekannte Messmethode etabliert sich damit allgemein zur Untersuchung von Flüssigkeiten. Neben der mit der Durchflusszytometrie messbaren Eigenschaften wie Anzahl und Aktivität von Bakterien ist auch die allgemeine Trübung von Interesse. Dieser Trübungswert wird traditionell mit einem eigens dafür vorgesehenen Gerät gemessen. Als nachteilig hat sich erwiesen, dass auch bei der Verwendung bekannter Messgeräte auf Basis der Durchflusszytometrie die Trübung bisher separat gemessen werden musste, da ein herkömmliches Durchflusszytometer die Trübung nicht bestimmen kann. Trübungsmessgeräte sind im direkten Vergleich mit einem Durchflusszytometer wesentlich einfacher und damit auch wirtschaftlicher. Wenn aber eine Durchflusszytometeranordnung bzw. ein Durchflusszytometer ohnehin im Einsatz ist, kann mit dem erfindungsgemäßen Verfahren aus den Messdaten der Durchflusszytometrie auch auf die Trübung der untersuchten Flüssigkeit geschlossen werden. Dabei können insbesondere Messdaten in Rohform, also vor jeglichen Korrekturen durch eine Signalverarbeitung, verwendet werden. Es ist möglich, dass bei konkreten Implementierungen Synergieeffekte der Signalverarbeitung verwendet werden können. Hier wird das eigentliche Verfahren bzw. die Methode beschrieben. Die Nutzung von Synergieeffekten ist anwendungsspezifisch.

Historisch wurde die Trübung insbesondere mit einem manuellen Transmissionstest eruiert. Dabei wurde die Erkennbarkeit von Ziffern hinter einer bestimmten Menge der zu bewertenden Flüssigkeit als Indikator verwendet. Dieses Verfahren wurde durch standardisierte Trübungseinheiten (zum Beispiel FAU, FNU, FTU, NTU, TE/E und EBC) ersetzt. Die einzige Flüssigkeit, bei der diese Standards äquivalente Ergebnisse zeigen, ist die Flüssigkeit Formazin. Bei realen Proben ist mit Abweichungen, zum Teil mit großen Abweichungen, zwischen den verschiedenen Standards zu rechnen. Die mit der hier vorgestellten Methode bzw. dem hier vorgestellten Verfahren ermittelten Messwerte entsprechen aufgrund der Eigenschaften des verwendeten Messsystems keinem der genannten Standards. Aufgrund der in der Durchflusszytometrie häufig verwendeten Wellenlänge der Lichtquelle von 488 nm (beispielsweise bei einem Laser) gegenüber der in der Spezifikation ISO-7027 festgelegten Wellenlänge kann das hierin beschriebene Verfahren nicht ohne weiteres das gesamte Spektrum an Flüssigkeiten abdecken. Mit geeigneten Gewichtungsparametern ist es aber möglich, die Resultate dem gewünschten Messstandard anzugleichen.

Das erfindungsgemäße Verfahren ist je nach Verfügbarkeit der Kanäle des Durchflusszytometers bzw. der Durchflusszytometeranordnung entweder mit dem Forward-Scatter oder dem Side-Scatter anzuwenden. Es ist auch möglich, beide Kanäle auszuwerten, um den Bereich, in welchem der ermittelte Wert einem Standard entspricht, zu erweitern.

Im Abschnitt "Adaptive Implementation" wird beschrieben, wie die Gewichtungsparameter aufgrund der Eingangsgrößen angepasst werden können, um den einem Standardverfahren äquivalenten Wertebereich zu vergrößern. Dies kann wiederum einer speziellen Implementierung des Grundverfahrens entsprechen.

Eine Steuer- und/oder Auswertesoftware weist bevorzugt Algorithmen auf, die eine Berechnung der entsprechenden Parameter optional erlauben.

Ein weiteres hierin beschriebenes Verfahren, welches beispielsweise mit der hierin beschriebenen Durchflusszytometeranordnung verwendet werden kann, jedoch auch einen eigenständigen Erfindungsaspekt darstellen kann, stellt die Möglichkeit zur Verfügung, Zellen im Zustand der Zellteilung bei Auswertung von flusszytometrischen Messdaten zu erkennen. Dieses Verfahren wird im Zusammenhang mit der zuvor beschriebenen Durchflusszytometeranordnung beschrieben, weist jedoch durchaus eigenständige erfinderische Bedeutung auf. Eine Übertragung des Verfahrens auf in anderen durchflusszytometrischen Anwendungen aufgefundene Messdaten ist von der Erfindung ebenfalls umfasst.

Die Durchflusszytometrie ist im medizinischen Sektor unter anderem auch bei Blutuntersuchungen weit verbreitet und auch seit Jahrzehnten etabliert. Dabei werden meist Partikel in Flüssigkeiten untersucht, die 2 µm und größer sind. Neuere Sensoren sowie feinere und schnellere Signalabtaster erreichen bei modernen Durchflusszytometern eine Messgenauigkeit, die es erlaubt, die zum Teil wesentlich kleineren Bakterien zu erfassen. Dies wird vermehrt in der Wasserqualitätsmessung genutzt, indem man die Bakterienkulturen, die im Wasser leben, aufzeichnet. Eine solche Messung stellt einen fixen Zustand zum gemessenen Zeitpunkt dar. Es lässt sich ohne weitere Informationen weder eine Aussage über die Vergangenheit noch über den zukünftigen Zustand des Wassers machen.

Bakterien vermehren sich asexuell entsprechend dem Prinzip der Zellteilung, beispielsweise durch Querteilung oder Knospung. Die Rate der Teilung hängt dabei vor allem von Umgebungsfaktoren wie Temperatur und Nährstoffangebot bzw. Nährstoffverfügbarkeit ab. Die optimale Temperatur hängt vom jeweiligen konkreten Bakterientyp ab. Ebenfalls ist eine benötigte Art des Nährstoffs bakteriumspezifisch. Da ein Durchflusszytometer bislang nicht dazu in der Lage ist, den Typ einer gemessenen Kolonie zu eruieren, ist es somit auch nicht möglich, festzustellen, ob eine konkrete Temperatur förderlich oder hemmend ist. Das Nachweisen von Nährstoffen in einer Flüssigkeit ist ein komplexer Prozess und damit weit außerhalb der Möglichkeiten bekannter Durchflusszytometer. Dennoch enthält eine Probe wie beispielsweise Wasser mit einer gewissen Wahrscheinlichkeit eine gewisse Anzahl an Bakterien, die sich gerade im Stadium der Teilung befinden.

Es soll somit ein Durchflusszytometer bzw. eine Durchflusszytometeranordnung zur Verfügung gestellt werden, womit insbesondere Zeit und Intensität ausreichend fein aufgelöst werden können, um mit einem Verfahren, welches als eigenständiger Erfindungsaspekt betrachtet werden kann, Bakterien in der Phase der Zellteilung zu erkennen. Dazu werden die Daten nach Maxima und Minima untersucht. Wenn zwei Maxima dicht aufeinanderfolgen und der mittige Minimalpunkt eine gewisse Tiefe erreicht, ist die Chance groß, dass es sich dabei um zwei Zellkerne handelt, welche von einer in der Teilung befindlichen Zelle stammen. Das Erkennen dieses spezifischen Merkmals hat zweierlei Nutzen.

Wird zur besseren Detailauflösung die Fläche eines Impulses integriert und zur Partikel-Identifikation verwendet, ergibt sich bei zwei aufeinanderfolgenden Signalpulsen eine leichte Verfälschung. Dieser Flächenfehler kann korrigiert werden.

Die Anzahl der Partikel im Stadium der Zellteilung wird den allgemein erkannten Partikeln gegenübergestellt. Daraus ergibt sich ein Bioaktivitäts-Faktor. Dieser Faktor kann sowohl über alle Partikel als auch Cluster-spezifisch erfolgen und ist letztlich Sache der Auswertung. Die Feststellung des Bioaktivitätsfaktors lässt Rückschlüsse auf vorhandene Nährstoffe, aber auch auf Sauerstoff zu.

Die zu messende Flüssigkeit kann insbesondere zu einem dünnen Strahl geformt werden. Dieser Strahl kann mit einer anregenden Lichtquelle, insbesondere mit einem Laser, beschossen werden. Die in der Flüssigkeit befindlichen Partikel reagieren entsprechend ihren optischen Eigenschaften mit der Lichtquelle und ergeben ein messbares optisches Muster.

Zur Messung des durch die Partikel generierten Signals können insbesondere optoelektronische Sensoren wie beispielsweise Photodioden oder Photomultiplier eingesetzt werden. Dies gilt nicht nur bei dem hier konkret beschriebenen Verfahren, sondern ganz allgemein. Die Photodetektoren erzeugen ein elektrisches Signal, welches digital gewandelt werden kann. Die Wandlung erfolgt dabei bevorzugt so schnell, dass der Abstand zwischen den Zellkernen möglichst akkurat beurteilt werden kann. Das digitale Signal wird kontinuierlich auf lokale Maximalwerte wie beispielsweise Peaks untersucht. Gleichzeitig bzw. spätestens nach Erkennen eines lokalen Maximums wird das Signal nach lokalen Minimalstellen untersucht.

Unterschreitet der Abstand zwischen zwei lokalen Maximalstellen eine gewisse Grenze, so ist dies ein Indiz dafür, dass die Pulse nicht unabhängig sind. Zur Beurteilung, ob es sich wirklich um eine Zelle im Teilungsstadium handelt, können weitere Parameter herangezogen werden. Die verwendbaren Parameter hängen dabei auch von der Genauigkeit des Messsystems ab. Zur Verbesserung kann der zulässige Unterschied der beiden Maxima eingeschränkt werden. Da die Zellkerne vom gleichen Typ sind, sollte die gemessen Größe der lokalen Maxima beinahe identisch sein. Es sind zwar diverse Szenarien denkbar, dass gleiche Zellkerne unterschiedliche Maxima ergeben (False Negative), im Allgemeinen ist aber davon auszugehen, dass die gemessene Amplitude im gleichen Größenbereich liegt.

Ein weiteres Indiz für eine Abhängigkeit von zwei aufeinanderfolgenden Pulsen ist die Höhe des eingeschlossenen Minimalwerts. Dieser wird mit den umgebenden Maximalwerten ins Verhältnis gesetzt. Ist das Minus zu groß, sprich beinahe äquivalent mit den einschließenden Maxima, deutet dies auf eine zu geringe Teilung hin und ist damit ein Ausschlusskriterium.

Es sei erwähnt, dass die Zellteilung typischerweise nur in einem bestimmten Zeitfenster der Zellteilung erkannt werden kann. Dies wird bevorzugt bei der Bestimmung eines Aktivitätsindex berücksichtigt. Weiter zu berücksichtigen sind geometrische Faktoren bzw. Abbildungsfehler. All diese Fehlerquellen, die im Allgemeinen zu einer niedrigeren Aktivitätszahl führen, können mit einem für das Messsystem zu bestimmenden Faktor kompensiert werden. Damit ergibt sich ein Aktivitätsindex, der mit einer Wahrscheinlichkeitsverteilung behaftet ist. Diese Verteilung kann sich stark von der Normalverteilung unterscheiden. Je nach Einsatzzweck kann diese Verteilung auch vernachlässigt werden.

In diesem Zusammenhang wird insbesondere darauf hingewiesen, dass alle im Bezug auf die Vorrichtung beschriebenen Merkmale und Eigenschaften aber auch Verfahrensweisen sinngemäß auch bezüglich der Formulierung des erfindungsgemäßen Verfahrens übertragbar und im Sinne der Erfindung einsetzbar und als mitoffenbart gelten. Gleiches gilt auch in umgekehrter Richtung, das bedeutet, nur im Bezug auf das Verfahren genannte, bauliche also vorrichtungsgemäße Merkmale können auch im Rahmen der Vorrichtungsansprüche berücksichtigt und beansprucht werden und zählen ebenfalls zur Offenbarung.

Weitere Merkmale und Vorteile wird der Fachmann den nachfolgend mit Bezug auf die beigefügte Zeichnung beschriebenen Ausführungsbeispielen entnehmen. Dabei zeigen:
Fig. 1: eine Durchflusszytometeranordnung gemäß einem Ausführungsbeispiel der Erfindung,
Fig. 2 und 3: eine Detektoranordnung,
Fig. 4 bis 7: eine Doppelschlitz-Streulichtblende,
Fig. 8 bis 11: eine Lasereinheit,
Fig. 12 bis 15:eine Durchflussmesszelle,
Fig. 16 und 17:eine Spritzeneinheit,
Fig. 18: ein allgemeines Funktionsprinzip,
Fig. 19: unverarbeitete Messdaten eines Streukanals eines Durchflusszytometers,
Fig. 20 und 21: schematisch eine mathematische Verknüpfung der aus dem Datenstrom erhobenen Parameter,
Fig. 22: schematisch den Ablauf einer typischen Zellteilung,
Fig. 23: schematisch die Aufzeichnung einer Signalform beim Abtasten einer in Teilung befindlichen Zelle,
Fig. 24 bis 28: einen Überstromfilter,
Fig. 29 bis 32: eine Richtoptik und
Fig. 33 und 34: schematisiert den Betrieb von Spritzeneinheiten.

In den Figuren sind gleiche oder einander entsprechende Elemente jeweils mit den gleichen Bezugszeichen bezeichnet und werden daher, sofern nicht zweckmäßig, nicht erneut beschrieben. Die in der gesamten Beschreibung enthaltenen Offenbarungen sind sinngemäß auf gleiche Teile mit gleichen Bezugszeichen bzw. gleichen Bauteilbezeichnungen übertragbar. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiterhin können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

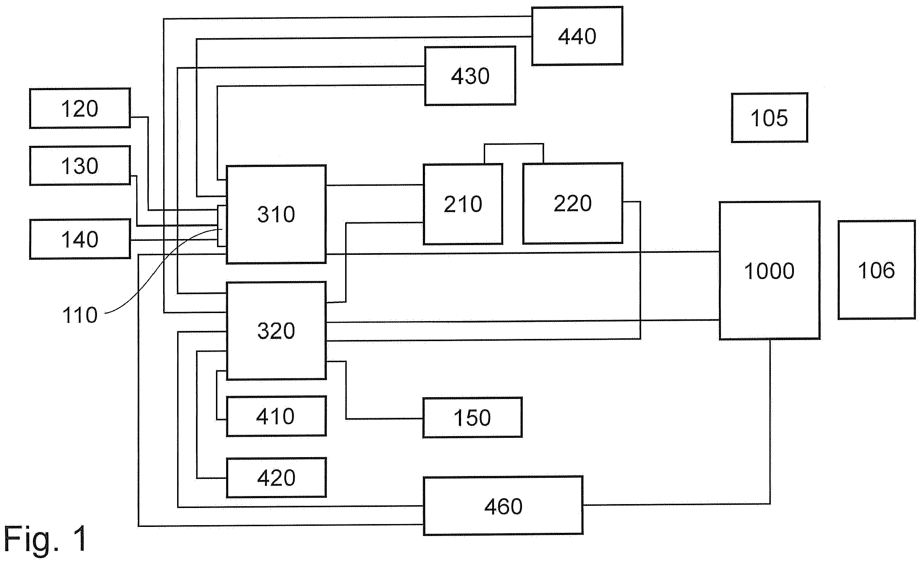

Fig. 1 zeigt schematisch eine Durchflusszytometeranordnung 100 gemäß einem Ausführungsbeispiel der Erfindung.

Die Durchflusszytometeranordnung 100 weist eine Detektoranordnung 1000 auf, welche dazu ausgebildet ist, eine Probenflüssigkeit zu analysieren und insbesondere darin befindliche Zellen zu detektieren. Dieser zugeordnet ist eine Auswerteeinrichtung 106.

Die Durchflusszytometeranordnung 100 weist einen Eingangsanschluss 110 auf, welcher hier als ein Element zu verstehen ist, an welchem zu analysierende Probenflüssigkeit aufgenommen wird. Daran angeschlossen sind eine ersten Entnahmeeinheit 120 und eine zweite Entnahmeeinheit 130, welche als jeweilige Überstromfilter ausgebildet sind. Diese sind dazu vorgesehen, Probenflüssigkeit aus einem kontinuierlichen Strom von Wasser, beispielsweise aus einer Wasserversorgung, zu entnehmen. Auf deren genaue Ausbildung wird weiter unten näher eingegangen werden. Des Weiteren ist an dem Eingangsanschluss 110 ein Einzelprobeneinlauf 140 angeordnet, mittels welchem in Einzelfällen einzelne Proben dem Eingangsanschluss 110 zugeführt werden können.

Die Durchflusszytometeranordnung 100 weist einen Mischer 210 und einen diesem nachgelagerten Inkubator 220 auf.

Mittels des Mischers 210 kann Probenflüssigkeit mit Farbstoff gemischt werden. Das dadurch entstehende Gemisch wird dann an den Inkubator 220 abgegeben, wobei der Inkubator 220 dazu ausgebildet ist, für entsprechende Bedingungen zu sorgen, damit der Farbstoff in die vorhandenen Zellen eingebaut wird und insbesondere an einer DNA oder RNA andockt. Hierzu ist der Inkubator 220 dazu ausgebildet, das von dem Mischer 210 gelieferte Gemisch für eine Zeit von 5 Minuten auf eine Temperatur von 40 °C aufzuwärmen. Es sei jedoch verstanden, dass hier grundsätzlich auch andere Kombinationen von Zeit und Temperatur möglich sind.

Um den Mischer 210 und den Inkubator 220 zu versorgen und Gemisch davon abzuziehen, weist die Durchflusszytometeranordnung 100 eine erste Pumpe 310 und eine zweite Pumpe 320 auf.

Die erste Pumpe 310 ist eingangsseitig mit dem Eingangsanschluss 110 verbunden. Ausgangsseitig ist sie mit dem Mischer 210 verbunden. Die erste Pumpe 310 kann somit dazu verwendet werden, Probenflüssigkeit an den Mischer 210 zu liefern.

Die zweite Pumpe 320 dient zum Liefern von Farbstoff. Hierzu weist die Durchflusszytometeranordnung 100 ein erstes Farbstoffreservoir 410 und ein zweites Farbstoffreservoir 420 auf, welche jeweils mit der zweiten Pumpe 320 verbunden sind. Ausgangsseitig ist die zweite Pumpe 320 mit dem Mischer 210 verbunden.

Die zweite Pumpe 320 dient auch dem Ansaugen von Gemisch aus dem Inkubator 220 und dem Liefern dieses Gemisches an die Detektoranordnung 1000. Hierzu ist die zweite Pumpe 320 eingangsseitig mit dem Inkubator 220 verbunden. Ausgangsseitig ist die zweite Pumpe 320 auch mit der Detektoranordnung 1000 verbunden, um einen Probenstrahl in einer weiter unten beschriebenen Durchflussmesszelle der Detektoranordnung 1000 zu erzeugen. Die Verbindungen der zweiten Pumpe 320 zum Mischer 210 und zur Detektoranordnung 1000 sind dabei schaltbar, so dass ausgewählt werden kann, ob die zweite Pumpe 320 in den Mischer 210 oder in die Detektoranordnung 1000 pumpt. Ebenso sind auch die Verbindungen zum Inkubator 220 und zu den Farbstoffreservoirs 410, 420 jeweils schaltbar, so dass ausgewählt werden kann, ob die zweite Pumpe 320 von dem ersten Farbstoffreservoir 410, von dem zweiten Farbstoffreservoir 420 oder von dem Inkubator 220 ansaugt.

Die Durchflusszytometeranordnung 100 weist des Weiteren einen Sekundäreingang 150 auf, welcher mit der zweiten Pumpe 320 schaltbar verbunden ist und das Liefern von einzelnen Proben an die zweite Pumpe 320 erlaubt. Somit kann grundsätzlich die gezeigte und beschriebene Vorbereitung von Proben bzw. von Gemisch umgangen werden und direkt eine Flüssigkeit in die zweite Pumpe 320 geleitet werden, welche unmittelbar an die Detektoranordnung 1000 zum Auswerten geliefert werden soll. Ebenso kann das Gemisch dort ausgegeben werden.

Die erste Pumpe 310 dient des Weiteren dem Liefern von Hüllflüssigkeit. Hierzu weist die Durchflusszytometeranordnung 100 ein Hüllflüssigkeitsreservoir 430 auf, in welchem Hüllflüssigkeit gelagert wird. Die erste Pumpe 310 ist eingangsseitig mit dem Hüllflüssigkeitsreservoir 430 verbunden und kann somit Hüllflüssigkeit aus dem Hüllflüssigkeitsreservoir 430 ansaugen. Die Verbindungen zum Eingangsanschluss 110 und zum Hüllflüssigkeitsreservoir 430 sind dabei jeweils schaltbar, so dass ausgewählt werden kann, ob die erste Pumpe 310 von dem Eingangsanschluss 110 oder von dem Hüllflüssigkeitsreservoir 430 aus ansaugen soll. Ausgangsseitig ist die erste Pumpe 310 auch mit der Detektoranordnung 1000 verbunden, um einen Hüllstrahl in der bereits erwähnten, in Fig. 1 nicht dargestellten Durchflussmesszelle zu erzeugen. Dabei sind die Verbindungen der ersten Pumpe 310 zum Mischer 210 und zur Detektoranordnung 1000 jeweils schaltbar ausgeführt, so dass ausgewählt werden kann, ob die erste Pumpe 310 zum Mischer 210 oder zur Detektoranordnung 1000 pumpen soll.

Die Durchflusszytometeranordnung 100 weist ferner eine Reinigungsflüssigkeitsversorgungsvorrichtung 440 auf. Diese ist mit den Pumpen 310, 320 schaltbar verbunden. Die Reinigungsflüssigkeitsversorgungsvorrichtung 440 ist dabei zum Liefern von Reinstwasser ausgebildet. Alternativ oder zusätzlich kann sie beispielsweise zum Liefern von chloriertem Wasser ausgebildet sein. Somit können die Pumpen 310, 320 sowie daran angeschlossene Komponenten bei Bedarf mit Reinstwasser und/oder mit chloriertem Wasser gereinigt werden, um eventuell vorhandene Bakterien oder andere Verschmutzungen zu entfernen.

Die Durchflusszytometeranordnung 100 weist ferner einen Entsorgungstank 460 auf, welcher mit den Pumpen 310, 320 verbunden ist. Außerdem ist er mit der Detektoranordnung 1000 verbunden. In dem Entsorgungstank 460 können nicht mehr benötigte Flüssigkeiten und insbesondere auch Flüssigkeiten, welche zum Spülen und Reinigen verwendet wurden, gelagert werden. Der Entsorgungstank 460 kann regelmäßig vorschriftsgemäß entsorgt werden.

Die Durchflusszytometeranordnung 100 weist ferner eine Steuerungseinrichtung 105 auf, welche hier ebenfalls nur schematisch dargestellt ist. Die Steuerungseinrichtung 105 ist dazu ausgebildet, die Pumpen 310, 320 zu steuern und auch die erwähnten bzw. sonst vorhandenen, in Fig. 1 nicht dargestellten Ventile zu schalten. Die Steuerungseinrichtung 105 kann somit den Betrieb der gesamten Durchflusszytometeranordnung 100 steuern.

Nachfolgend wird ein typischer Betrieb der Durchflusszytometeranordnung 100 beispielhaft beschrieben.

Zunächst wird die erste Pumpe 310 eingangsseitig geöffnet und Probenflüssigkeit aus dem Eingangsanschluss 110 angesaugt. Diese Probenflüssigkeit kann entweder aus einer der Entnahmeeinheiten 120, 130 oder aus dem Einzelprobeneinlauf 140 stammen. Ebenso wird mittels der zweiten Pumpe 320 eine definierte Menge Farbstoff aus den Farbstoffreservoirs 410, 420 angesaugt. Dies kann entweder gleichzeitig oder zeitlich versetzt erfolgen.

Anschließend werden die beiden ersten und zweiten Pumpen 310, 320 ausgangsseitig geöffnet, und zwar zu dem Mischer 210 hin. Die Verbindungen zu der Detektoranordnung 1000 werden dabei geschlossen. Somit werden Probenflüssigkeit und Farbstoff in den Mischer 210 gepumpt.

Die beiden ersten und zweiten Pumpen 310, 320 werden dann so betätigt, dass sie gleichzeitig Probenflüssigkeit und Farbstoff in den Mischer 210 abgeben. Dies erfolgt synchron, so dass Probenflüssigkeit und Farbstoff gleichmäßig in den Mischer 210 strömen.

Der Mischer 210 ist so ausgelegt, dass er für eine gründliche Durchmischung der gelieferten Probenflüssigkeit mit dem gelieferten Farbstoff sorgt. Das dabei entstehende Gemisch wird dann an den Inkubator 220 abgegeben. Dieser erwärmt das Gemisch für eine Zeit von 5 Minuten auf eine Temperatur von 40 °C.

Das derart präparierte Gemisch wird dann von der zweiten Pumpe 320 angesaugt. Hierzu wird die zweite Pumpe 320 eingangsseitig zu dem Inkubator 220 geöffnet und entsprechend betätigt.

Während des Ansaugens des Gemisches durch die zweite Pumpe 320 wird die erste Pumpe 310 synchron derart betrieben, dass in der Kombination aus Mischer 210 und Inkubator 220 ein konstanter Druck herrscht. Dies beugt Beschädigungen vor.

Das entsprechend bearbeitete Gemisch befindet sich nunmehr in der zweiten Pumpe 320. Um dies auswerten zu können, ist ferner Hüllflüssigkeit erforderlich. Diese wird von der ersten Pumpe 310 aus dem Hüllflüssigkeitsreservoir 430 angesaugt.

Zum Analysieren werden sowohl das Gemisch wie auch die Hüllflüssigkeit gleichzeitig in die Detektoranordnung 1000 gepumpt. Hierzu werden die zweite Pumpe 320 und die erste Pumpe 310 ausgangsseitig geöffnet und so betätigt, dass das Gemisch und die Hüllflüssigkeit synchron in die Detektoranordnung 1000 gelangen. Das Gemisch bildet dabei einen Probenstrahl durch die bereits erwähnte Durchflussküvette, während die Hüllflüssigkeit einen Hüllflüssigkeitsstrahl durch diese Probenküvette erzeugt. Mittels dieser Hüllflüssigkeit kann somit der Probenstrahl hydrodynamisch fokussiert werden. Darauf wird weiter unten näher eingegangen werden.

Nach diesem Vorgang kann, falls erforderlich, eine Reinigung der Pumpen 310, 320 oder auch anderer Komponenten mittels Reinstwasser und/oder chloriertem Wassers erfolgen. Dabei eventuell anfallende verunreinigte Flüssigkeiten oder auch andere nicht mehr benötigte Flüssigkeiten können in dem Entsorgungstank 460 entsorgt werden.

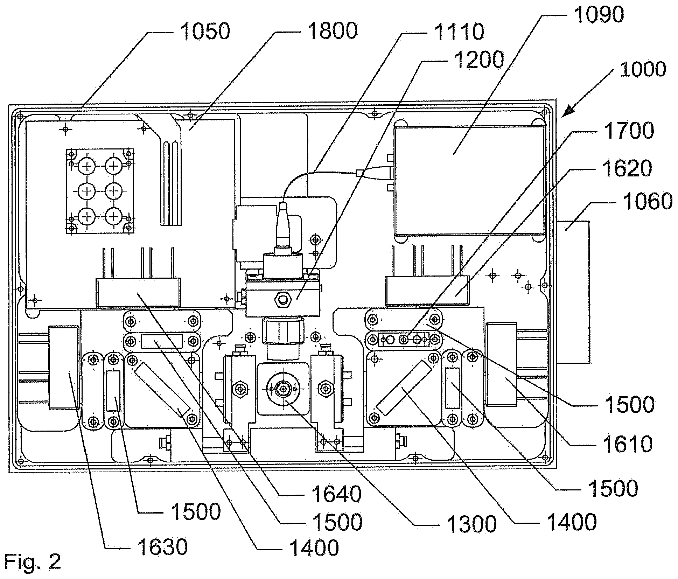

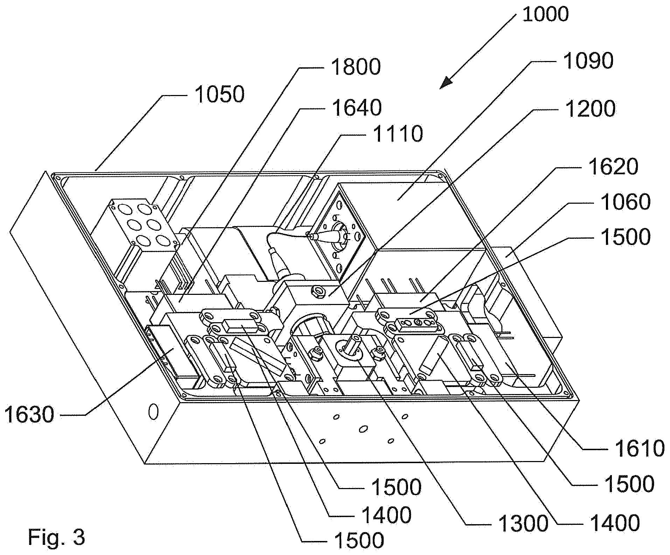

Die Fig. 2 und 3 zeigen die Detektoranordnung 1000 in größerer Detailliertheit.

Die Detektoranordnung 1000 ist in einem Gehäuse 1050 enthalten, welches als Monoblock bzw. einstückig ausgeführt ist. Das Gehäuse 1050 ist dabei aus einem Vollmaterial, beispielsweise durch Fräsen, ausgebildet. Es weist vorliegend an einer seiner Schmalseiten elektrische Anschlüsse 1060 auf.

In dem Gehäuse 1050 befindet sich eine Laseranordnung 1090, welche weiter unten näher beschrieben werden wird.

Die Detektoranordnung 1000 weist eine Durchflussmesszelle 1300 auf, auf welche ebenfalls weiter unten näher eingegangen werden wird. Dieser vorgeschaltet ist eine Richtoptik 1200, auf welche ebenfalls weiter unten näher eingegangen werden wird. Zwischen der Laseranordnung 1090 und der Richtoptik 1200 ist eine lichtleitende Faser 1110 vorgesehen, über welche ein von der Lasereinheit 1090 erzeugter Laserstrahl zu der Richtoptik 1200 geleitet wird. Die Richtoptik 1200 richtet und fokussiert den Laserstrahl in definierter Weise auf die Durchflussmesszelle 1300, durch welche der bereits mit Bezug auf Fig. 1 erwähnte Probenstrahl sowie die Hüllflüssigkeit strömen. Die Hüllflüssigkeit dient dabei zur Fokussierung des Probenstrahls auf einen Durchmesser von etwa 30 µm.

Durch die in dem Probenstrahl enthaltenen Bakterien, welche mit dem bereits weiter oben erwähnten Farbstoff markiert sind, wird der Laserstrahl seitlich gestreut. Ebenso wird der Laserstrahl an sich an der Durchflussmesszelle 1300 seitlich gestreut. Das gestreute Licht wird durch zwei Strahlteiler 1400, welche bezüglich des Laserstrahls seitlich zur Durchflussmesszelle 1300 angeordnet sind, aufgeteilt. Bei diesen Strahlteilern 1400 handelt es sich vorliegend um Strahlteiler, welche nicht wellenlängensensitiv ausgeführt sind.

Durch die Strahlteiler 1400 wird das gestreute Licht auf insgesamt vier Detektoren 1610, 1620, 1630, 1640 geleitet. Vor dreien dieser Detektoren, nämlich den Detektoren 1610, 1630, 1640 sind jeweilige Bandpassfilter 1500 vorgeschaltet, welche einen jeweiligen geringen Teil des optischen Spektrums ausfiltern, so dass der jeweilige Detektor 1610, 1630, 1640 nur den entsprechenden Teil des optischen Spektrums detektiert. Dies ermöglicht eine wellenlängenselektive Detektion. Es kann auch der jeweilige Strahlteiler 1400 als Bandpassfilter ausgebildet sein.

Vor einem der Detektoren, nämlich dem Detektor 1620, ist eine Doppelspalt-Streulichtblende vorgeschaltet, auf welche weiter unten näher eingegangen werden wird. Diese blendet den Ursprungs-Laserstrahl, welcher an der Durchflussmesszelle 1300 an sich reflektiert wird, aus. Der Doppelspalt-Streulichtblende 1700 ist ein Bandpassfilter mit einer Anregungswellenlänge von 488 nm nachgeschaltet. Dies ermöglicht es, auch die Wellenlänge des Laserstrahls, welche vorliegend bei 488 nm liegt, zur Detektion mittels des Detektors 1620 zu verwenden.

Des Weiteren weist die Detektoranordnung 1000 eine Auswerteelektronik 1800 auf, welche für eine erste Verarbeitung der durch die Detektoren 1610, 1620, 1630, 1640 gewonnenen Daten sorgt.

Die Detektoren 1610, 1620, 1630, 1640 weisen jeweilige Transimpedanzverstärker auf, was die Auswertung der gewonnenen Signale erleichtert.

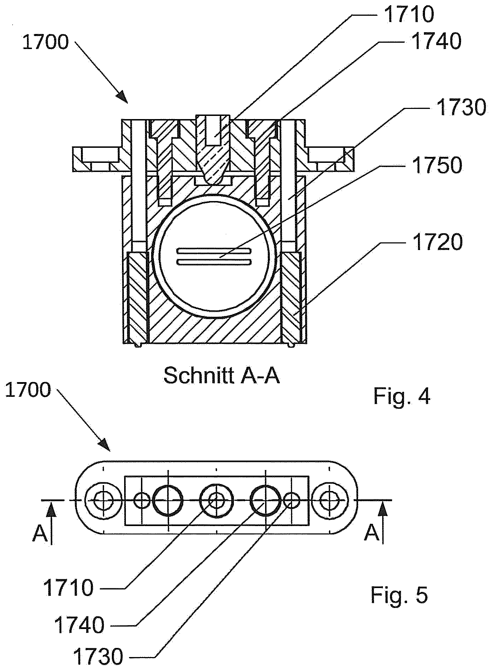

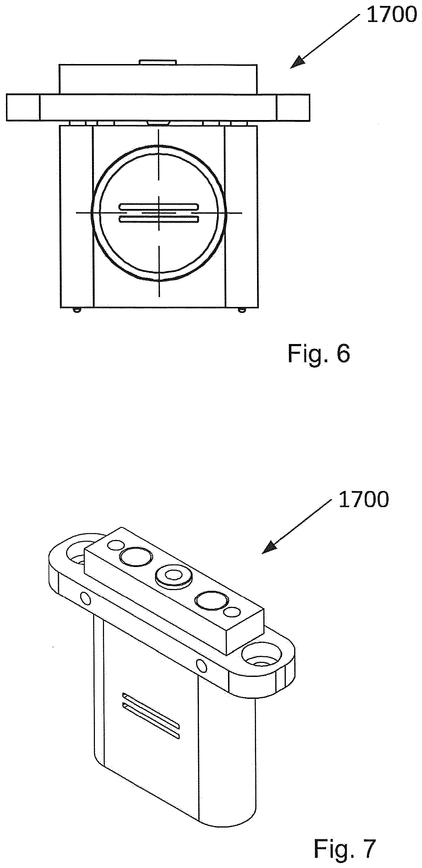

Die Fig. 4 bis 7 zeigen die Doppelspalt-Streulichtblende 1700. Diese ermöglicht es, in der Durchflusszytometrie das seitlich und vorwärts gestreute Licht so auf einen jeweiligen Detektor treffen zu lassen, dass ein statischer Signal-Offset unterdrückt wird und das gestreute Licht, verursacht durch Partikel oder Bakterien, ohne Beeinträchtigung der Signalqualität auf den jeweiligen Detektor trifft. Vorliegend handelt es sich hierbei um den bereits beschriebenen Detektor 1620.

Die Doppelschlitz-Streulichtblende 1700 kann mittels einer integrierten, vertikalen Führung über Führungsstifte 1730, die mit Druckfederstiften 1720 in Eingriff stehen, exakt in die Höhe des Lichtstrahls bzw. über eine Feststell- bzw. Arretierungsschraube 1740 sowie eine Feinstellschraube 1710 justiert werden, um ein optimales Nutzsignal zu erhalten und Störungen durch Streulicht auszuschließen. Hierzu kann der reflektierte Ursprungs-Laserstrahl auf einen Steg 1750 gerichtet werden.

Vorteilhaft ist mit der Doppelschlitz-Streulichtblende 1700 nahezu die gesamte Bandbreite der optischen Messsignale nutzbar, denn direkt in die optischen Detektoren reflektiertes Licht wird vermieden.

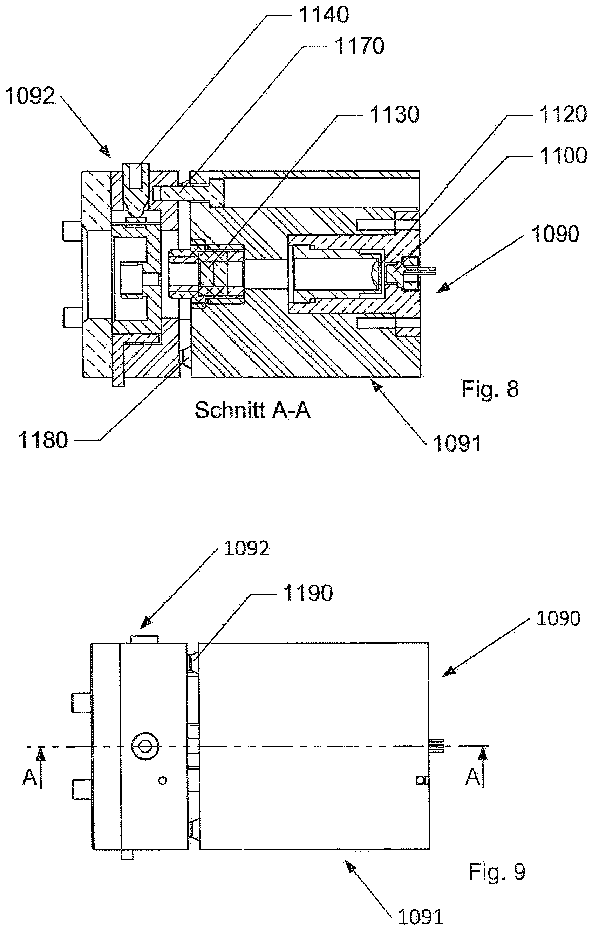

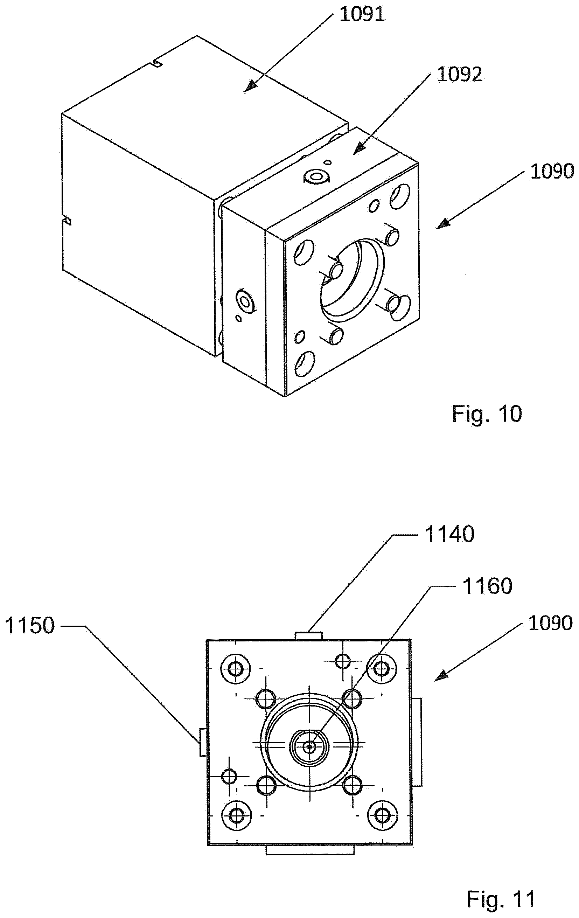

Die Fig. 8 bis 11 zeigen die Lasereinheit 1090 in weiterer Detailliertheit und unterschiedlichen Ansichten.

Die Lasereinheit 1090 teilt sich auf in einen Laserabschnitt 1091 mit integrierter Faserkopplung und eine laterale Faserpositioniereinheit bzw. Einkopplungseinheit 1092.

Die Lasereinheit 1090 weist eine Laserdiode 1100 auf, welche einen Laserstrahl mit einer Wellenlänge von 488 nm erzeugt. Dieser nachgeordnet sind eine Kollimationslinse 1120, eine Fokussierlinse 1130 und die bereits erwähnte laterale Faserpositioniereinheit bzw. Einkopplungseinheit 1092. Eine Justierung ist mittels einer ersten Feinstellschraube 1140 in y-Richtung und einer zweiten Feinstellschraube 1150 in x-Richtung möglich. Die Zuführung der bereits erwähnten Faser 1110 wird über einen Faserstecker 1160 realisiert.

Die Einkopplungseinheit 1092 wird über zwei Fixierschrauben 1170 verschraubt.

Über eine dritte Feinstellschraube 1180 kann eine Verkippung in x-Richtung erreicht werden. Über eine vierte Feinstellschraube 1190 kann eine Verkippung in y-Richtung erreicht werden.

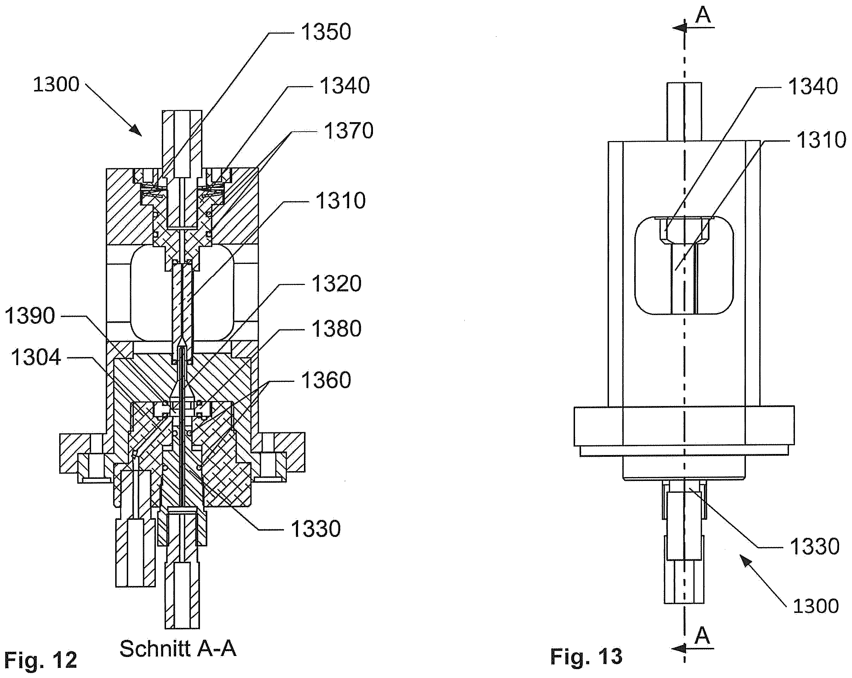

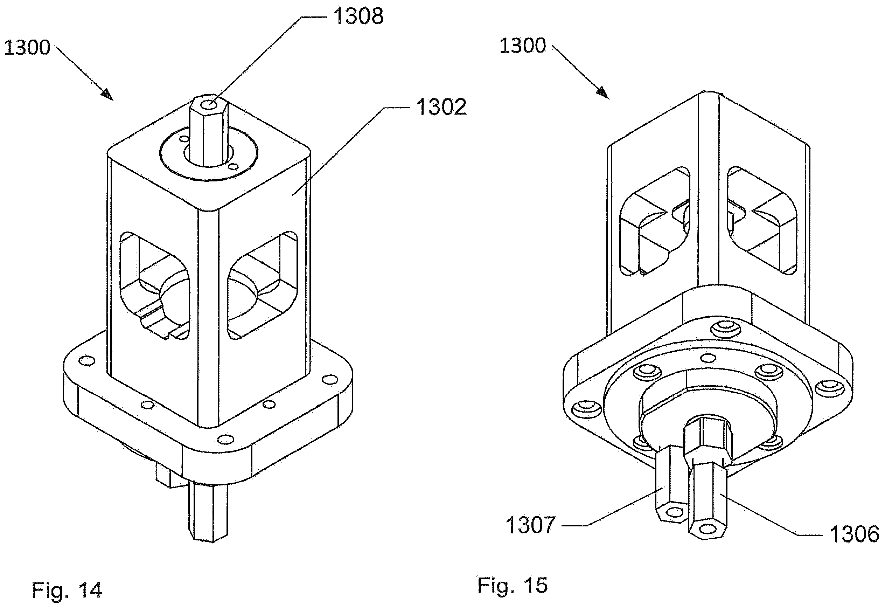

Die Fig. 12 bis 15 zeigen die Durchflussmesszelle 1300 in größerer Detailliertheit. Diese beinhaltet insbesondere eine Zuführung von Probenflüssigkeit und Hüllflüssigkeit. Diese werden in einer Quarzglasküvette zusammengeführt.

Die Durchflussmesszelle 1300, welche auch als Durchflussküvette bezeichnet werden kann, stellt sicher, dass die Zellen einer Probenflüssigkeit einzeln und immer im Zentrum (im optischen Fokus) durchgeschleust werden. Die geometrischen Abmessungen und Verhältnisse, insbesondere einer Proben- und Hüllstromzuführung, beeinflussen den Probenstrahldurchmesser, die Hüllstrommenge und die Stabilität des Probenstrahls.

Der Probenstrahl wird über eine Probenkanüle 1320 zugeführt. Eine Justage dieser Probenkanüle 1320 soll nicht nur bei der Montage der Einheit vorgenommen werden können, sondern auch während des Betriebs.

Eine Probenzuführung kann während einer Kalibrationsmessung vertikal positioniert werden, indem eine Nadel zur Aufgabe einer Probe mit einer lateral positionierten, geführten Probennadelfassung gehalten wird. Die Probennadelfassung 1330 weist ein Außengewinde auf, welches in ein Gegengewinde eingreift. Dadurch kann eine einfache Verstellung in vertikaler Richtung vorgenommen werden, indem die Probennadelfassung 1330 gedreht wird. Seitlich zur Probennadelfassung 1330 sind zwei O-Ringe 1360 vorgesehen, welche für eine Abdichtung und Fixierung sorgen. Diese wirken auch als mechanische Bremse, um nach erfolgter Justierung ein Dejustieren zu verhindern, welches beispielsweise durch Vibrationen verursacht sein kann. Außerdem wirken sie dichtend.

Bei dem erwähnten Außengewinde handelt es sich vorliegend um ein Feingewinde. Dies ermöglicht eine besonders exakte Einstellung.

Die Hüllflüssigkeit wird an einer Stelle über einen Hüllstromflüssigkeitskanal 1304 in einen zirkularen Verteilring 1390 eingeleitet. Die Hüllflüssigkeit soll die Probenkanüle 1320 und den Probenstrahl homogen umströmen. Hierfür ist insbesondere eine Lochblende 1380 vorgesehen, durch welche die Hüllflüssigkeit geradegerückt wird.

Die Durchflussmesszelle 1310 umfasst ferner einen Gegenhalter 1340, eine Druckfeder 1350 und zwei obere O-Ringe 1370 am Gegenhalter 1340 zur Fixierung der Küvette. Über den Hüllstromflüssigkeitskanal 1304 wird der Hüllstrahl geleitet. Über einen Auslass 1308 werden Probe und Hüllflüssigkeit bzw. weitere Medien nach Abschluss der Messung abgeführt.

Eine Zuführung der Probe erfolgt von unten über eine unterseits der Einheit vorgesehene Probenzuführung 1306. Dieser zugeordnet ist eine Hüllstromflüssigkeitszuführung 1307.

Die gesamte Einheit ist mechanisch stabil umschlossen und gelagert in einem Support 1302. Dieser kann in dem bereits erwähnten Gehäuse 1050, welches insbesondere als Monoblock ausgeführt sein kann, angeordnet sein und als Einheit mit allen Bestandteilen, welche eben beschrieben wurden, beispielsweise zum Austausch oder zum Reinigen entnommen werden.

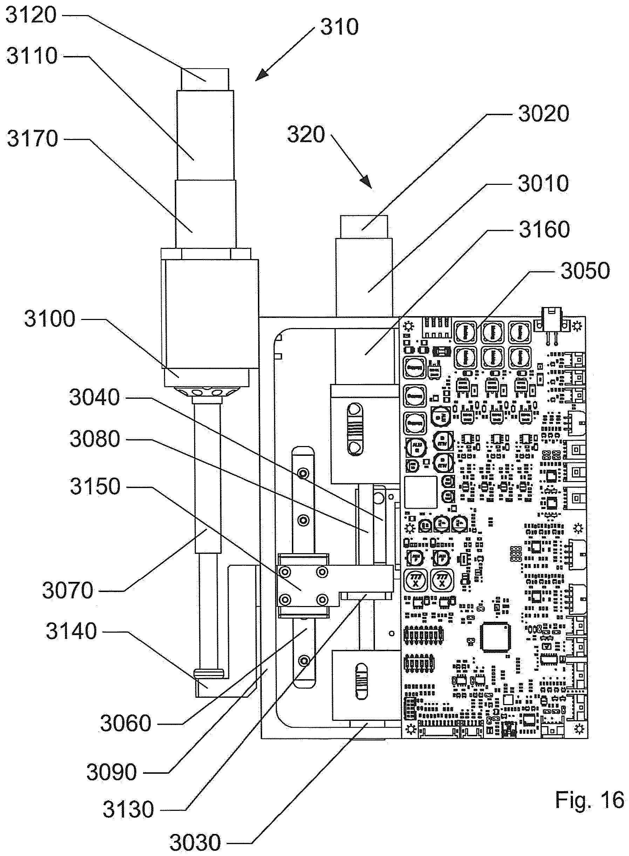

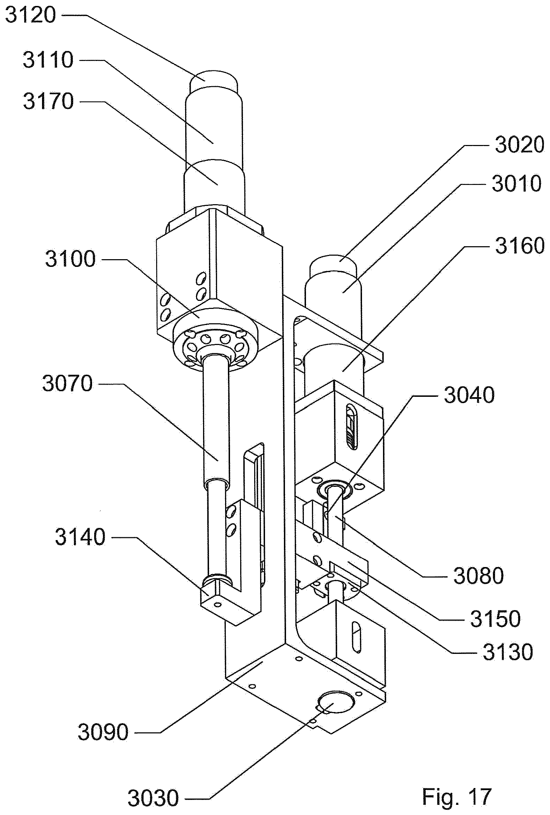

Die Fig. 16 und 17 zeigen eine Ausführungsform einer Spritzeneinheit. Diese kann beispielsweise zur Steuerung der bereits mit Bezug auf Fig. 1 erwähnten Pumpen 310, 320 verwendet werden. Vorliegend ist sie dementsprechend gezeigt.

Zum Antrieb der Pumpen 310, 320 dienen Motoren 3010, 3110. Diese zugeordnet sind jeweilige Motoren-Encoder 3020, 3120, ein Positions-Encoder 3030 sowie ein Referenzsensor 3040. Den Motoren bzw. Spritzen sind jeweils einzelne unabhängige Motorsteuerungen 3050 zugeordnet, welche vorliegend jedoch softwareseitig verbunden sind.

Jede Spritzeneinheit weist eine Linearführung 3060 und eine Spritze 3070 mit zugeordneter Antriebsspindel 3080 auf. Zur Anordnung der jeweiligen Spritzeneinheit in einem Gerät verfügt diese über einen Spritzensupport 3090, der auch die übrigen Elemente trägt.

Der Spritzeneinheit zur Steuerung des Medienflusses zugeordnet ist ein Rotationsventil 3100, welches über einen eigenen Motor 3110 und einen eigenen Motoren-Encoder 3120 verfügt.

Einem Spritzenarm 3140 zugeordnet ist eine Mitnahmemutter 3130, welche in Verbindung mit einem Linearschlitten 3150 steht. Über ein Planetengetriebe 3160 kann die jeweilige Spritze bzw. über ein weiteres Planetengetriebe 3170 das Rotationsventil bewegt werden.

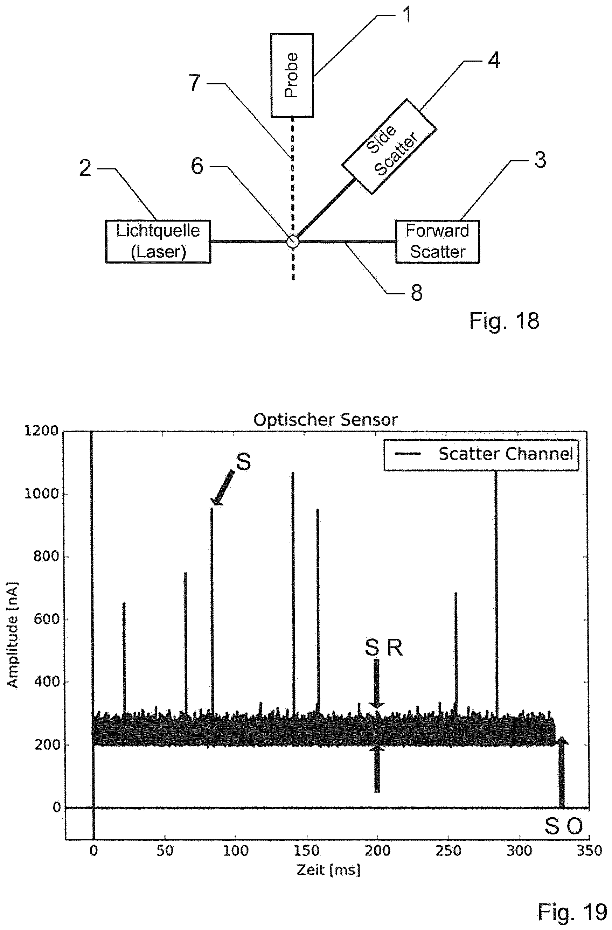

Fig. 18 zeigt schematisch eine vereinfachte Darstellung des allgemeinen Funktionsprinzips der Durchflusszytometrie.

Dabei wird grundsätzlich eine Probe 1 zu einem Probenstrahl 7 geformt. Dieser Strahl wird von einer Lichtquelle 2 bestrahlt. Diese ist vorliegend beispielsweise ein Laser. Trifft der Lichtstrahl auf ein Partikel 6, so ergibt sich auf einem vorwärts gerichteten Sensor 3 eine Abschattung, während seitwärts ein Streulicht 8 auf einen Side-Scatter-Sensor 4 fällt.

Die Fig. 19 zeigt beispielhaft unverarbeitete Messdaten eines Streukanals eines Durchflusszytometers. Aus diesen Messdaten können Kenngrößen berechnet werden, welche mit entsprechender Gewichtung zur Berechnung der Trübung verwendbar sind. Der Graph zeigt das elektrische Signal, welches digital gewandelt wurde, im vorliegenden Fall als Stromsignal.

Die genaue Art des Signals ist dabei unerheblich bzw. ist vom eingesetzten Sensor abhängig. Gängige Ausgangsgröße eines optoelektronischen Sensors ist ein elektrischer Strom.

Mit SO ist dabei ein allgemeiner Signal-Offset bezeichnet. Mit SR ist ein Signalrauschen bezeichnet. Mit S ist ein Signal bezeichnet, welches durch Partikel verursacht wird, also das allgemeine Nutzsignal.

Die Graphik der Fig. 19 zeigt insgesamt sieben Partikel, die sich klar aus dem Signalrauschen abheben. Es ist möglich, dass viele kleinere Partikel im Rauschen untergehen. Diese können mit der Verwendung des Rauschens als eigenständige Größe berücksichtigt werden.

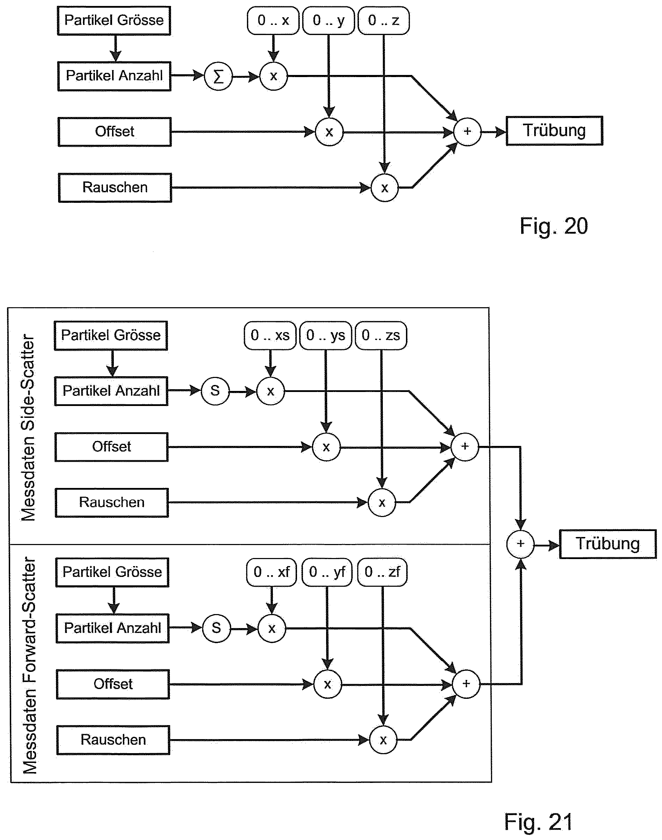

Die Fig. 20 und 21 zeigen eine beispielhafte mathematische Verknüpfung der aus dem Datenstrom erhobenen Parameter gemäß einem Ausführungsbeispiel eines erfindungsgemäßen Verfahrens. Abhängig von den Eigenschaften eines Messsystems und eines Messwandlers werden Parameter gewichtet. Dabei kann einem Gewichtungsfaktor auch der Wert null zugewiesen werden, was einer faktischen Ignoranz des entsprechenden Parameters entspricht.

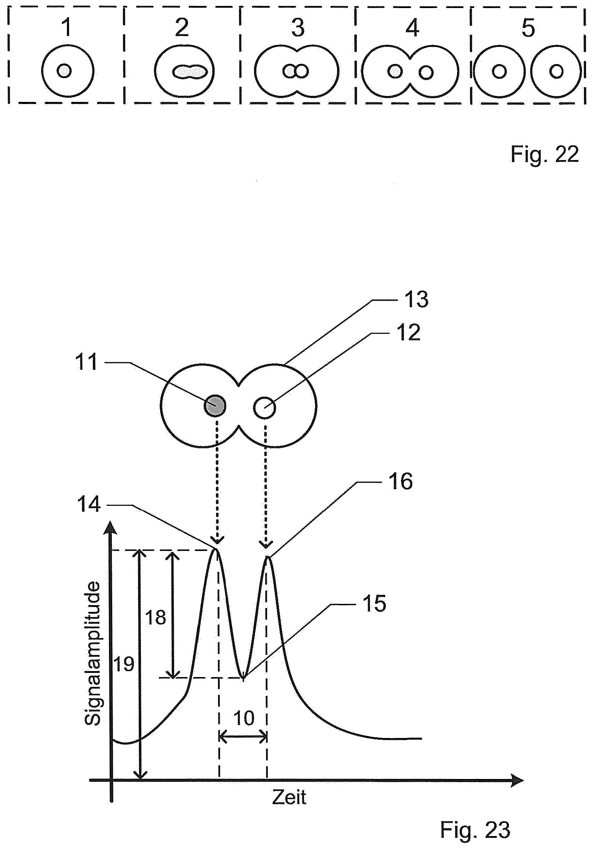

Die Fig. 22 zeigt schematisch einen typischen Ablauf einer Zellteilung. Die mit Nummern 1 bis 5 dargestellten Stationen werden nachfolgend kurz beschrieben:
1: Einzelne gesunde Zelle mit genügend Nährstoffen in unmittelbarer Umgebung;
2: Zelle, deren Zellkern sich zu duplizieren beginnt;
3: Zelle mit zwei vollständig ausgebildeten Zellkernen, Hülle beginnt sich durch Einschnürung zu teilen;
4: Kerne berühren sich nicht mehr, umgebende Zelle schließt sich;
5: zwei vollständige und eigenständige Zellen.

Die Fig. 23 zeigt beispielhaft eine Aufzeichnung einer Signalform beim Abtasten einer in Teilung befindlichen Zelle. Dabei sind zwei Zellkerne 11, 12 zu erkennen. Diese haben noch eine gemeinsame Zellwand 13.

Ein erstes Maximum 14 im Signalverlauf wird durch den ersten Zellkern 11 verursacht. Ein zweites Maximum 16 wird durch den zweiten Zellkern 12 verursacht. Ein Minimum 15 zwischen den beiden Maxima 14, 16 ist abhängig vom Abstand zwischen den beiden Zellkernen 11, 12.

Nach dem zweiten Maximum 16 zeigt sich ein abflachender Verlauf. Für das hier vorgestellte Beispiel wird angenommen, dass der nächste Puls so weit entfernt liegt, dass keine weitere Beeinflussung zu diesem Zeitpunkt besteht.

Der Abstand zwischen Maximum und Minimum ist mit Bezugszeichen 18 bezeichnet. Dieser Parameter wird für die hier vorgestellte Methode als Eingangsvariable verwendet. Ebenfalls kann die Signalamplitude 19 zum Zeitpunkt des ersten Maximums 14 verwendet werden. Alternativ könnte hierzu auch die Signalamplitude des zweiten Maximums 16 verwendet werden. Der zeitliche Signal-Abstand zwischen den beiden Zellkernen 11, 12 ist mit Bezugszeichen 10 bezeichnet. Auch hierbei handelt es sich um einen Eingangsparameter.

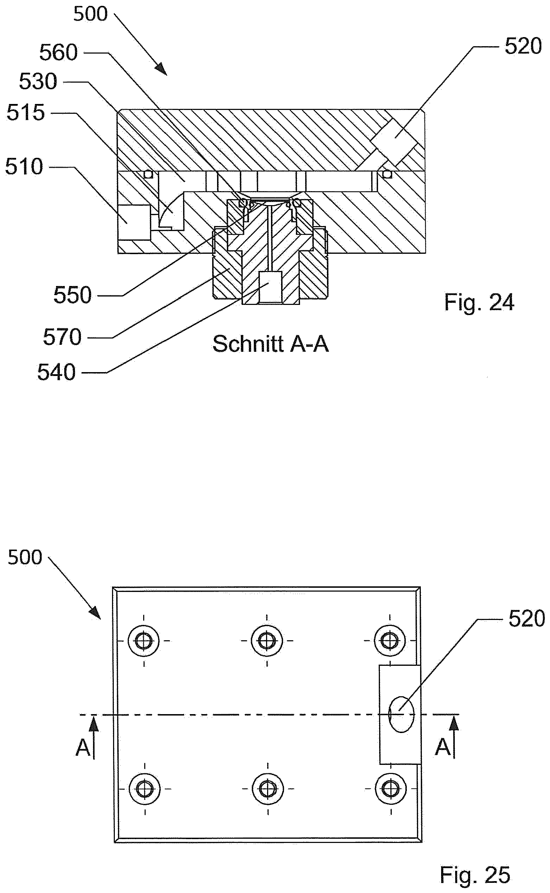

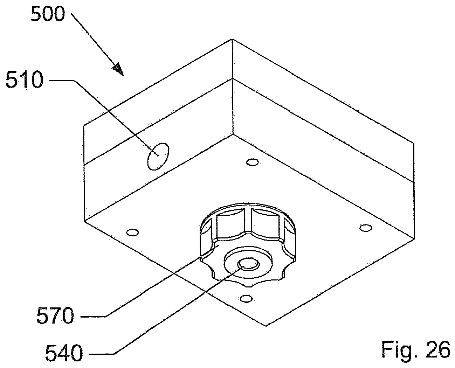

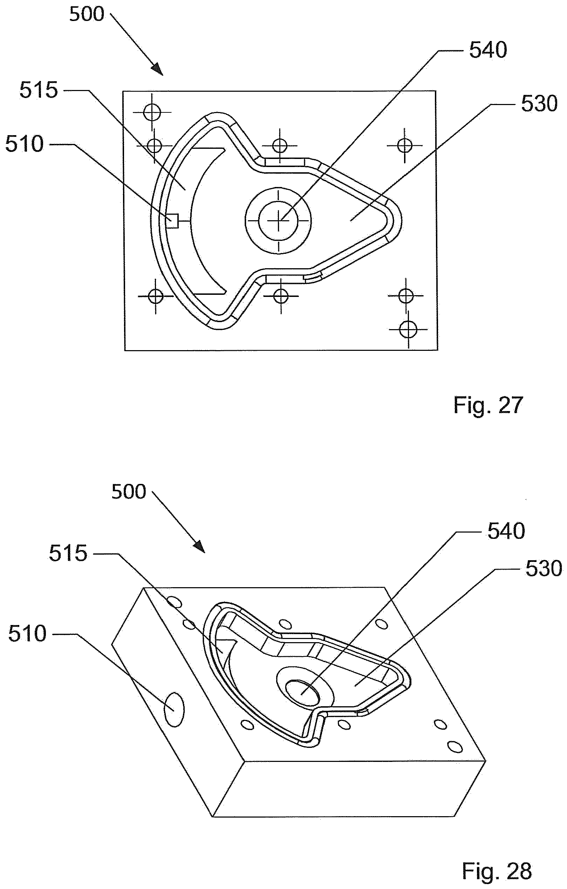

Die Fig. 24 bis 28 zeigen einen bereits mit Bezug auf Fig. 1 erwähnten Überstromfilter 500. Dieser dient dazu, eine blasenfreie Probenentnahme von Fluiden in Probendurchflusssystemen zu ermöglichen, was für die Durchflusszytometrie von zentraler Bedeutung ist. Blasen, wie auch Mikroblasen, verursacht durch die Probenentnahme oder durch das Überströmen von Probenentnahmefiltern (Turbulenz), können zu Messfehlern führen. Beispielsweise können Streulichteffekte, laterale Verschiebungen des Probenstrahls sowie bei Mikroblasen Störungen im Streulichtkanal verursacht werden.

Der hier gezeigte Überstromfilter 500 ermöglicht eine äußerst blasenfreie Gewinnung von wässrigen Proben. Durch ein speziell ausgelegtes Design und die Anordnung der weiter unten beschriebenen Komponenten, insbesondere eines Kanals, sowie des Einlass- und Auslassanschlusses, wird erreicht, dass sich gelöster Sauerstoff und Blasen am Überstromfilter sammeln und nicht in die Messprobe gelangen. Außerdem ist ein einfacher Ersatz des Filters möglich.

Der Überstromfilter 500 weist einen Kanal 530 auf, welcher Einlass 510 und einen Auslass 520 aufweist. Durch diesen Kanal 530 strömt somit ein Fluid, insbesondere ein Wasser aus einer üblichen Trinkwasserversorgung, wobei das Wasser vom Einlass 510 zum Auslass 520 strömt.

Angrenzend an den Kanal 530 ist ein Filter 550 vorgesehen, welcher wiederum mit einem Probenauslass 540 verbunden ist. Der Probenauslass 540 ist mit dem in Fig. 1 dargestellten Eingangsanschluss 110 verbunden.

Der Kanal 530 ist im Bereich des Filters 550 vom Einlass 510 zum Auslass 520 hin verjüngt ausgeführt. Dies erlaubt eine Erhöhung der Strömungsgeschwindigkeit, was Blasenbildung vermeidet und einen Abzug entstehender Blasen ermöglicht. Der Kanal 530 weist am Einlass 510 einen Einlasstrichter 515 auf, mittels welchem einströmende Flüssigkeit über den Kanal 530 besser verteilt wird. Auch dies vermeidet Blasenbildung

Der Auslass 520 ist zum Kanal 530 schräg ausgebildet, wobei der Auslass 520 in Einbaustellung nach oben weist. Dies ermöglicht einen unmittelbaren Abzug von Blasen nach oben, welche ja die Tendenz haben, im Wasser nach oben zu steigen.

Der Filter 550 ist von einer Dichtung 560 umgeben. Dies vermeidet eine Leckage des durch den Kanal 530 strömenden Wassers. Der Filter 530 wird von einer Mutter 570 gehalten, welche über ein nicht dargestelltes Gewinde einfach ein- und ausgeschraubt werden kann. Dies erlaubt eine zuverlässige Haltung des Filters 550 und ein einfaches Wechseln des Filters durch Entfernen der Mutter 570.

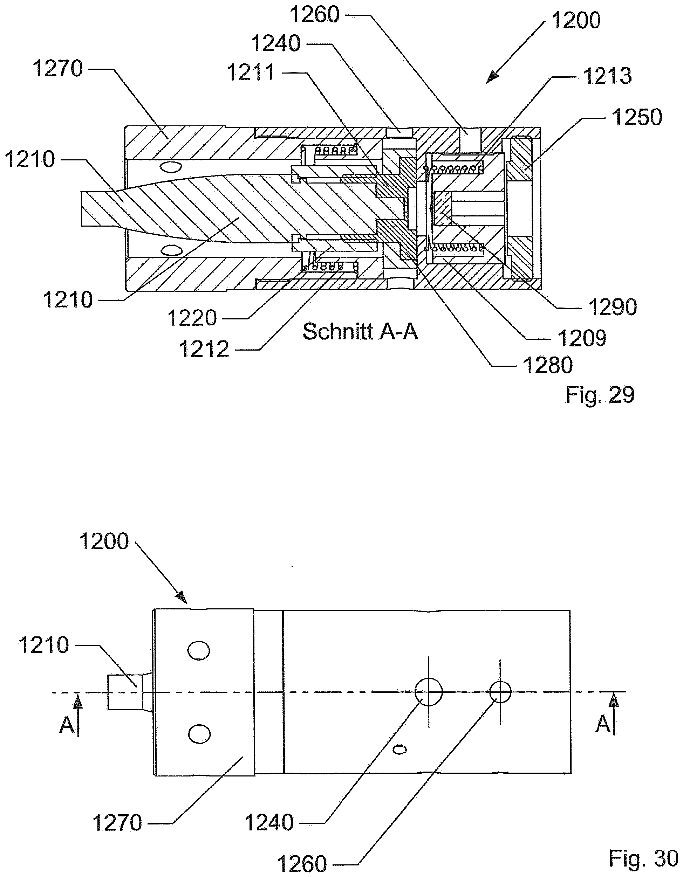

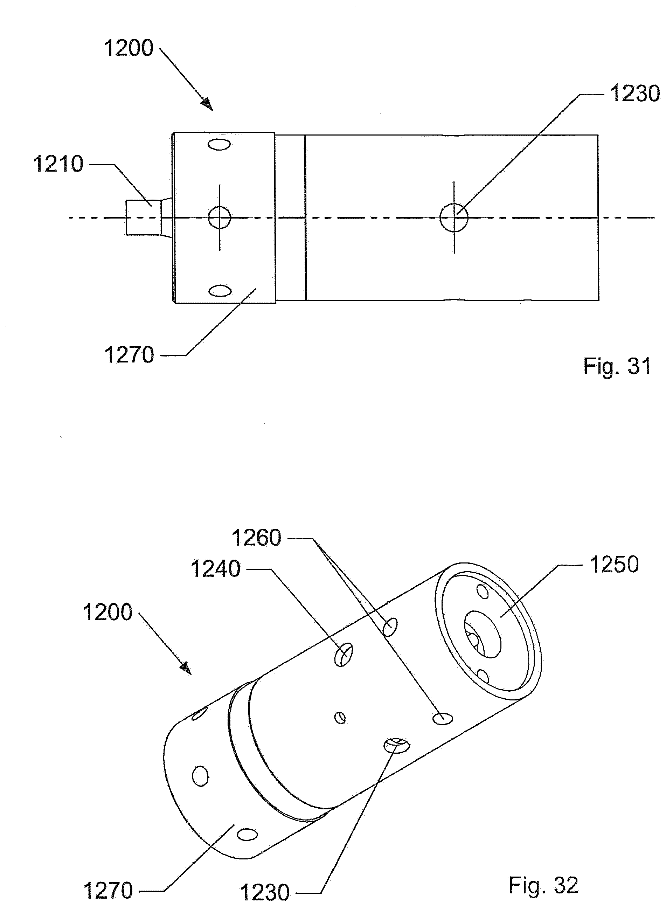

Die Fig. 29 bis 32 zeigen die Fokussieroptik 1200 in größerer Detailliertheit.

Die Lichteinkopplung in die Durchflussmesszelle 1300 ist eines der Kernelemente der Durchflusszytometrie. Je exakter und definierter der Laserstrahl die einzelnen, mit Fluoreszenzfarbe angefärbten Bakterien anleuchtet, desto geringer ist die Streuung der Leuchtintensität der Bakterien. Leuchtintensitätsschwankungen haben zur Folge, dass die Detektoren das Bakterium nicht korrekt erfassen, da die Leuchtintensität Informationen über die Größe des Bakteriums enthält.

Eine homogene Beleuchtungsform (Beleuchtungsfenster horizontal, vertikal (Flussrichtung in der Messküvette) und in die Tiefe ist Voraussetzung, um die Streuung gering zu halten.

Aus dem Stand der Technik bekannte lichtleitergekoppelte Strahlformerobjektive bestehen typischerweise aus einer Fokussierlinse, einem Strahlformerelement und einer Abbildungslinse. Dieser optische Aufbau mit drei Optikelementen ist konstruktiv aufwändig, etwa das Positionieren der Linsen. Außerdem erfordert eine solche Ausführung eine hohe mechanische Stabilität, welche außerhalb von definierten Laborumgebungen nicht gewährleistet werden kann.

Mittels der hier vorgestellten Fokussiereinheit 1200 wird es ermöglicht, einen Laserstrahl am Lichtaustritt eines Lichtleiters präzise zu justieren und mechanisch zu fixieren. Die Anforderungen an Umgebungsbedingungen werden dadurch deutlich verringert.

Die Fokussiereinheit 1200 weist dabei einen Lichtleiteranschluss 1210 auf. Sie weist ferner einen Lichtleiterstecker 1220 auf, welcher zum Halten des Lichtleiteranschlusses 1210 dient. Der Lichtleiterstecker 1220 kann mittels einer x-Positionierung 1230 und einer y-Positionierung 1240 in einer Ebene quer zum Lichtstrahl verstellt werden.

Ein Optikelement 1290 sorgt für die Kombination der Funktionalitäten einer Fokussierlinse, eines Strahlformerelements und einer Abbildungslinse. Es handelt sich hierbei somit um ein einziges Element, welches in sich stabil ist und gegen Einflüsse wie beispielsweise Vibrationen oder Temperaturschwankungen resistent ist. Das Optikelement 1290 wird durch eine Gewindescheibe 1250 gehalten. Eine Optikelementfassung kann mittels einer Zylinderschraube 1260 in x-Richtung gehalten werden. Des Weiteren ist eine Klemmvorrichtung 1270 vorgesehen, um für eine laterale Positionierung und Rotation zu sorgen.

Der Lichtleiterstecker 1220 wird durch einen Positionierflansch 1280 gehalten und fixiert.

Das Optikelement 1290 wird durch eine Optikelementfassung 1209 gehalten.

Des Weiteren ist ein Positionierring 1211 vorgesehen. Mittels einer Druckfeder 1212 kann eine Verschiebung in x-Richtung, y-Richtung und eine Rotation unterstützt werden.

Einer weiteren Druckfeder 1213 ist die Optikelementfassung 1209 in z-Richtung zugeordnet. Außerdem ist eine Blockierung 1260 der Optikelementfassung 1209 mittels zweier Zylinderschraube in z-Richtung vorgesehen.

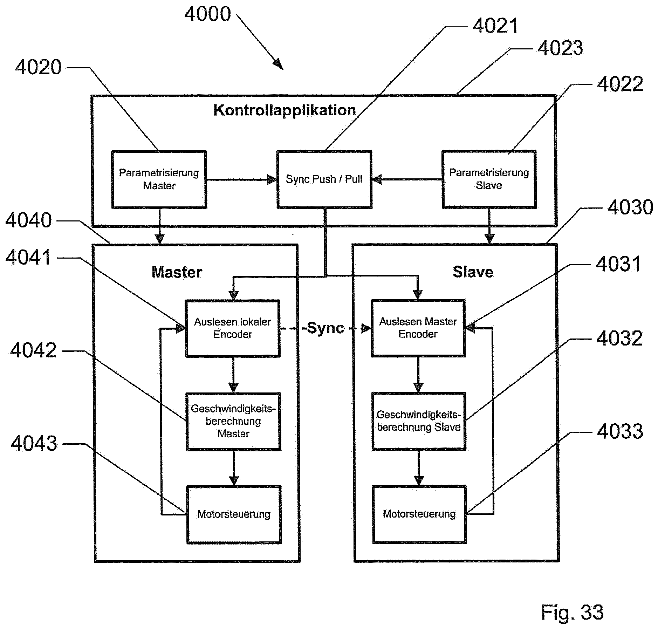

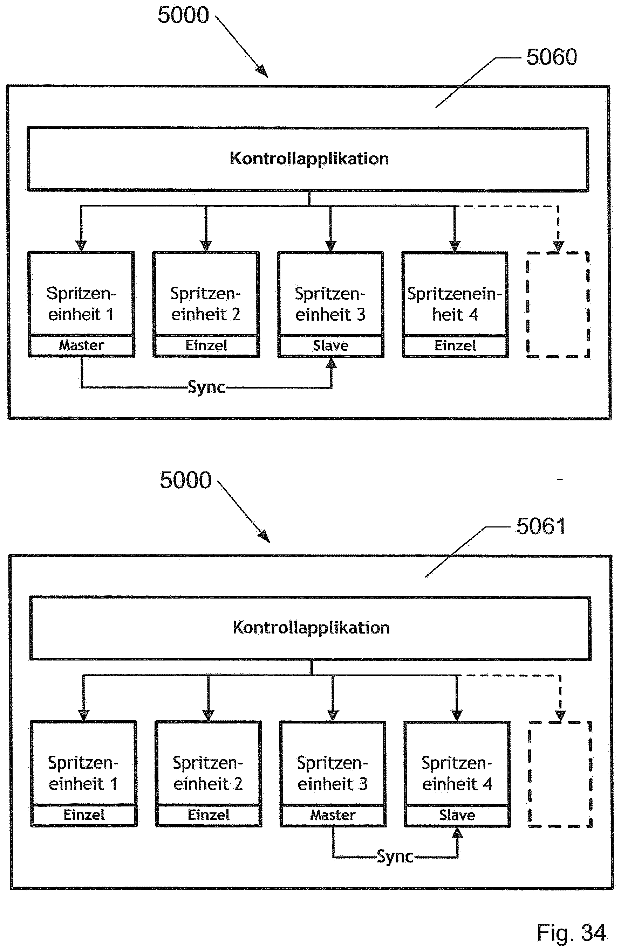

Die Fig. 33 und 34 zeigen schematisiert den Betrieb von Spritzeneinheiten in einem erfindungsgemäßen Durchflusszytometer. Hierzu sind Schemata 4000 bzw. 5000 dargestellt.

Der Einzelbetrieb einer Spritzeneinheit erfolgt durch Parametrisierung 4020 der notwendigen Informationen durch eine Kontrollapplikation 4023, welche mit der jeweiligen Spritzeneinheit durch einen Kommunikationskanal verbunden ist. Anschließend wird der Prozess (beispielsweise Aufziehen und Ausstoßen) durch die Kontrollapplikation gestartet (4021). Die auf der Spritzeneinheit laufende Steuerapplikation 4040 übersetzt das Volumen in eine Anzahl zu fahrender Inkremente eines Positions-Encoders auf der Spritzeneinheit. Zyklisch wird auf der Spritzeneinheit der aktuelle Wert des Positions-Encoders ausgelesen (4041), die zugehörige parametrisierte Geschwindigkeit berechnet (4042) und anschließend der Motorsteuerung die gewünschte Geschwindigkeit zugewiesen (4043). Dies geschieht, bis die gewünschte Encoder-Position erreicht wurde und somit das entsprechende Volumen aufgezogen bzw. ausgestoßen wurde.

Die Umsetzung der Erfindung für den synchronen Betrieb zweier oder mehrerer Spritzeneinheiten wird im Folgenden beschrieben: Das Positions-Encoder-Signal einer Spritzeneinheit (Master) 4040 wird elektrisch an eine zweite bzw. mehrere Spritzeneinheit(en) (Slave) 4030 gekoppelt. Für die Mastereinheit 4040 bleiben der Prozess des Aufziehens bzw. Ausstoßens identisch zum beschriebenen Einzelbetrieb. Die Kontrollapplikation 4023 parametrisiert (4022) die Slave-Einheiten 4030 mit dem zu bewegenden Volumen und einem berechneten Faktor, der auf die Encoder-Pulse des Masters 4040 angewendet wird. Dieser Faktor entspricht dem Verhältnis der Fluide in Abhängigkeit von den verwendeten Spritzenvolumina und der Gesamtzahl der Encoder-Pulse über eine komplette Spritzenlänge. Weiterhin wird die Slave-Einheit 4030 in einen Modus gesetzt, der die Steuerungsapplikation auf dem Slave 4030 in den Zustand versetzt, in welchem primär die Pulse des Master-Encoders verwendet werden.

Die Kontrollapplikation 4023 startet den synchronen Prozess 21 auf dem Master 4040. Der Slave 4030 liest zyklisch die Encoder-Pulse des Masters aus (4031), berechnet aus den Pulsen und dem parametrisierten Faktor ein fließendes Mittel der Geschwindigkeit (4032) und weist dieses der Motorsteuerung (4033) zu. Da die zu fahrende Anzahl von Inrementen des Slaves 4030 vorab parametrisiert wurde, wird zudem ständig überprüft, ob die finale Position beinahe erreicht wurde, sich also innerhalb einer definierten Anzahl von Inkrementen zum Ziel befindet. Trifft dies zu, so wird ab diesem Zeitpunkt nicht mehr der Master-Encoder ausgewertet, sondern der aktuelle eigene Positions-Encoder, um die endgültige Position zu erreichen. Der Prozess ist abgeschlossen, wenn beide Spritzeneinheiten ihre finale Encoder-Position erreicht haben.

Durch die Parametrisierung der Kontrollapplikation kann eine Spritzeneinheit entweder für sich alleine oder im synchronen Verbund betrieben werden, wobei sie beim synchronen Betrieb sowohl Master als auch Slave sein kann. Beispielhaft werden in einem Prozessschritt (5060) vier Spritzeneinheiten angesteuert. Die Spritzeneinheiten zwei und vier sind in diesem Prozessschritt im Einzelbetrieb. Spritzeneinheit eins und drei werden zum synchronen Betrieb parametrisiert, wobei Spritzeneinheit eins als Master fungiert und Spritzeneinheit drei als Slave dem Master folgt. In einem folgenden Prozessschritt (5061) mit physikalisch identischem Aufbau werden die Spritzeneinheiten nun so konfiguriert, dass die vormals als Slave operierende Spritzeneinheit drei nun als Master für Spritzeneinheit vier fungiert.

Es sei erwähnt, dass eine Abtastung von Signalpulsen der weiter oben erwähnten Detektoren bevorzugt mit 20 bis 60 Abtastungen pro Signalpuls erfolgen kann. Dies ermöglicht eine exakte Erkennung der Pulsform. Hierzu wird beispielsweise mit einer Auflösung zwischen 2 Megasamples pro Sekunde und 4 Megasamples pro Sekunde abgetastet.

Entsprechend ist bevorzugt ein Analog-Digital-Wandler (ADC) ausgebildet. Dieser kann beispielsweise eine Sampling-Rate zwischen 2 und 4 Megasamples pro Sekunde haben. Bevorzugt ist eine Signalauflösung von 24 Bit vorgesehen.

## Patentansprüche

1. Durchflusszytometeranordnung, aufweisend
- eine Durchflussmesszelle (1300),
- einen Mischer (210),
- eine erste Pumpe (310) und eine zweite Pumpe (320),
- einen Eingangsanschluss (110),
- ein Farbstoffreservoir (410, 420), und
- ein Hüllflüssigkeitsreservoir (430),
- wobei die erste Pumpe (310) eingangsseitig mit dem Eingangsanschluss (110) zum Ansaugen von Probenflüssigkeit verbunden ist,
- wobei die zweite Pumpe (320) eingangsseitig mit dem Farbstoffreservoir (410, 420) zum Ansaugen von Farbstoff verbunden ist,
- wobei die erste Pumpe (310) und die zweite Pumpe (320) ausgangsseitig mit dem Mischer (210) verbunden sind, um Probenflüssigkeit und Farbstoff in den Mischer (210) zu pumpen, und der Mischer (210) zum Mischen der Probenflüssigkeit und des Farbstoffs zu einem Gemisch ausgebildet ist,
- wobei die zweite Pumpe (320) eingangsseitig mit dem Mischer (210) zum Ansaugen des Gemischs verbunden ist, und
- wobei die zweite Pumpe (320) ausgangsseitig mit der Durchflussmesszelle (1300) verbunden ist, um aus dem Gemisch einen Probenstrahl durch die Durchflussmesszelle (1300) zu erzeugen,
**dadurch gekennzeichnet, dass**
- die erste Pumpe (310) eingangsseitig mit dem Hüllflüssigkeitsreservoir (430) zum Ansaugen von Hüllflüssigkeit verbunden ist, und
- die erste Pumpe (310) ausgangsseitig mit der Durchflussmesszelle (1300) verbunden ist, um aus der Hüllflüssigkeit einen Hüllstrahl durch die Durchflussmesszelle (1300) zu erzeugen, welcher den Probenstrahl umgibt.

2. Durchflusszytometeranordnung nach einem der vorhergehenden Ansprüche, **dadurch gekennzeichnet, dass**
- der Mischer (210) ausgangsseitig mit einem Inkubator (220) verbunden ist, welcher ausgangsseitig mit der zweiten Pumpe (320) verbunden ist.

3. Durchflusszytometeranordnung nach einem der vorhergehenden Ansprüche, **dadurch gekennzeichnet, dass**
- die erste Pumpe (310) dazu ausgebildet ist, ein vorgegebenes erstes Volumen an Probenflüssigkeit von dem Eingangsanschluss (110) anzusaugen,
- die zweite Pumpe (320) dazu ausgebildet ist, ein vorgegebenes zweites Volumen an Farbstoff von dem Farbstoffreservoir (410, 420) anzusaugen, und
- die erste Pumpe (310) und die zweite Pumpe (320) dazu ausgebildet sind, das erste Volumen an Probenflüssigkeit und das zweite Volumen an Farbstoff, zeitlich dem Ansaugen nachgelagert, gleichzeitig in den Mischer (210) zu pumpen.

4. Durchflusszytometeranordnung nach einem der vorhergehenden Ansprüche, **dadurch gekennzeichnet, dass**
- die Pumpen (310, 320) als spindelbetätigte Kolbenpumpen ausgeführt sind.

5. Durchflusszytometeranordnung nach einem der vorhergehenden Ansprüche, **dadurch gekennzeichnet, dass**
- die Durchflusszytometeranordnung (100) eine Detektoranordnung (1000) aufweist, welche wiederum folgendes aufweist:
- einen Laser (1100), welcher dazu ausgebildet ist, einen Laserstrahl zu erzeugen,
- eine Richtoptik (1200), welche dazu ausgebildet ist, den Laserstrahl auf die Durchflussmesszelle (1300) zu richten,
- eine Anzahl von Detektoren (1610, 1620, 1630, 1640), welche dazu ausgebildet sind, den durch die Durchflussmesszelle (1300) hindurchgetretenen Laserstrahl zu detektieren.

6. Durchflusszytometeranordnung nach einem der vorhergehenden Ansprüche, **dadurch gekennzeichnet, dass**
- die Durchflusszytometeranordnung (100) eine Steuerungseinrichtung (105) aufweist, welche dazu konfiguriert ist, die Pumpen (310, 320) zu steuern.

7. Durchflusszytometeranordnung nach Anspruch 6,
**dadurch gekennzeichnet, dass**
- die Steuerungseinrichtung (105) dazu konfiguriert ist, die erste Pumpe (310) und die zweite Pumpe (320) derart zu steuern, dass sie gleichzeitig vorgegebene Volumina an Probenflüssigkeit und Farbstoff in den Mischer (210) pumpen.

8. Durchflusszytometeranordnung nach einem der Ansprüche 6 oder 7, **dadurch gekennzeichnet, dass**
- die Steuerungseinrichtung (105) dazu konfiguriert ist, die zweite Pumpe (320) derart zu steuern, dass die zweite Pumpe (320) Gemisch aus dem Mischer (210) oder Inkubator (220) ansaugt.

9. Durchflusszytometeranordnung nach Anspruch 7 und Anspruch 8,
**dadurch gekennzeichnet, dass**
- die Steuerungseinrichtung (105) dazu konfiguriert ist, das Pumpen von Probenflüssigkeit und Farbstoff in den Mischer (210) zuerst zu veranlassen, anschließend eine vorbestimmte Inkubationszeit zu warten, und anschließend das Ansaugen des Gemischs zu veranlassen.

10. Durchflusszytometeranordnung nach einem der Ansprüche 6 bis 9, **dadurch gekennzeichnet, dass**
- die Steuerungseinrichtung (105) dazu konfiguriert ist, die erste Pumpe (310) so zu steuern, dass sie Hüllflüssigkeit in die Durchflussmesszelle (1300) pumpt, und gleichzeitig die zweite Pumpe (320) so zu steuern, dass sie aus dem Mischer (210) oder Inkubator (220) angesaugtes Gemisch in die Durchflussmesszelle (1300) pumpt.

## Claims

1. Flow cytometer arrangement comprising
- a flow measurement cell (1300),
- a mixer (210),
- a first pump (310) and a second pump (320),
- an inlet connection (110),
- a dye reservoir (410, 420), and
- a sheath fluid reservoir (430),
- wherein the first pump (310) is connected on the inlet side to the inlet connection (110) for suction of sample fluid,
- wherein the second pump (320) is connected on the inlet side to the dye reservoir (410, 420) for suction of dye,
- wherein the first pump (310) and the second pump (320) are connected on the outlet side to the mixer (210), in order to pump sample fluid and dye into the mixer (210), and the mixer (210) is designed to mix the sample fluid and the dye to give a mixture,
- wherein the second pump (320) is connected on the inlet side to the mixer (210) for suction of the mixture, and
- wherein the second pump (320) is connected on the outlet side to the flow measurement cell (1300) in order to produce a sample jet through the flow measurement cell (1300) from the mixture, **characterized in that**
- the first pump (310) is connected on the inlet side to the sheath fluid reservoir (430) for suction of sheath fluid,
- the first pump (310) is connected on the outlet side to the flow measurement cell (1300) in order to produce a sheath jet through the flow measurement cell (1300) that ensheaths the sample jet from the sheath fluid.

2. Flow cytometer arrangement according to any of the preceding claims, **characterized in that**
- the mixer (210) is connected on the outlet side to an incubator (220) which is connected on the outlet side to the second pump (320).

3. Flow cytometer arrangement according to any of the preceding claims, **characterized in that**
- the first pump (310) is designed to suck in a defined first volume of sample fluid from the inlet connection (110),
- the second pump (320) is designed to suck in a defined second volume of dye from the dye reservoir (410, 420), and
- the first pump (310) and the second pump (320) are designed to pump the first volume of sample fluid and the second volume of dye, after the time of suction, simultaneously into the mixer (210).

4. Flow cytometer arrangement according to any of the preceding claims, **characterized in that**
- the pumps (310, 320) are in the form of spindle-actuated piston pumps.

5. Flow cytometer arrangement according to any of the preceding claims, **characterized in that**
- the flow cytometer arrangement (100) comprises a detector arrangement (1000) which in turn comprises the following:
- a laser (1100) designed to produce a laser beam,
- directing optics (1200) designed to direct the laser beam toward the flow measurement cell (1300),
- a number of detectors (1610, 1620, 1630, 1640) designed to detect the laser beam that has passed through the flow measurement cell (1300).

6. Flow cytometer arrangement according to any of the preceding claims, **characterized in that**
- the flow cytometer arrangement (100) comprises a control device (105) configured to control the pumps (310, 320).

7. Flow cytometer arrangement according to Claim 6, **characterized in that**
- the control device (105) is configured to control the first pump (310) and the second pump (320) in such a way that they simultaneously pump defined volumes of sample fluid and dye into the mixer (210).

8. Flow cytometer arrangement according to either of Claims 6 and 7, **characterized in that**
- the control device (105) is configured to control the second pump (320) in such a way that the second pump (320) sucks mixture out of the mixer (210) or incubator (220) .

9. Flow cytometer arrangement according to Claim 7 and Claim 8, **characterized in that**
- the control device (105) is configured first to trigger the pumping of sample fluid and dye into the mixer (210), then to wait for a predetermined incubation time, and then to trigger the suction of the mixture.

10. Flow cytometer arrangement according to any of Claims 6 to 9, **characterized in that**
- the control device (105) is configured to control the first pump (310) such that it pumps sheath fluid into the flow measurement cell (1300), and simultaneously to control the second pump (320) such that it pumps mixture sucked in from the mixer (210) or incubator (220) into the flow measurement cell (1300).

## Revendications

1. Agencement de cytomètre en flux, comprenant :
- une cellule de mesure en flux (1300),
- un mélangeur (210),
- une première pompe (310) et une deuxième pompe (320),
- un raccord d'entrée (110),
- un réservoir de colorant (410, 420) et
- un réservoir de liquide d'enveloppe (430),
- la première pompe (310) étant reliée côté entrée au raccord d'entrée (110) pour aspirer l'échantillon liquide,
- la deuxième pompe (320) étant reliée côté entrée au réservoir de colorant (410, 420) pour aspirer le colorant,
- la première pompe (310) et la deuxième pompe (320) étant reliées côté sortie au mélangeur (210) pour pomper l'échantillon liquide et le colorant dans le mélangeur (210), et le mélangeur (210) étant configuré pour mélanger l'échantillon liquide et le colorant en un mélange,
- la deuxième pompe (320) étant reliée côté entrée au mélangeur (210) pour aspirer le mélange, et
- la deuxième pompe (320) étant reliée côté sortie à la cellule de mesure en flux (1300) pour produire à partir du mélange un jet d'échantillon à travers la cellule de mesure en flux (1300),
**caractérisé en ce que**
- la première pompe (310) est reliée côté entrée au réservoir de liquide d'enveloppe (430) pour aspirer le liquide d'enveloppe, et
- la première pompe (310) est reliée côté sortie à la cellule de mesure en flux (1300) pour produire à partir du liquide d'enveloppe un jet d'enveloppe à travers la cellule de mesure en flux (1300), qui entoure le jet d'échantillon.

2. Agencement de cytomètre en flux selon l'une quelconque des revendications précédentes, **caractérisé en ce que**
- le mélangeur (210) est relié côté sortie à un incubateur (220), qui est relié côté sortie à la deuxième pompe (320).

3. Agencement de cytomètre en flux selon l'une quelconque des revendications précédentes, **caractérisé en ce que**
- la première pompe (310) est configurée pour aspirer un premier volume prédéterminé d'échantillon liquide à partir du raccord d'entrée (110),
- la deuxième pompe (320) est configurée pour aspirer un deuxième volume prédéterminé de colorant à partir du réservoir de colorant (410, 420), et
- la première pompe (310) et la deuxième pompe (320) sont configurées pour pomper simultanément le premier volume d'échantillon liquide et le deuxième volume de colorant dans le mélangeur (210), en aval dans le temps de l'aspiration.

4. Agencement de cytomètre en flux selon l'une quelconque des revendications précédentes, **caractérisé en ce que**
- les pompes (310, 320) sont des pompes à piston actionnées par broche.

5. Agencement de cytomètre en flux selon l'une quelconque des revendications précédentes, **caractérisé en ce que**
- l'agencement de cytomètre en flux (100) comprend un agencement de détecteur (1000), qui comprend luimême les éléments suivants :
- un laser (1100), qui est configuré pour produire un faisceau laser,
- une optique directionnelle (1200), qui est configurée pour diriger le faisceau laser sur la cellule de mesure en flux (1300),
- un nombre de détecteurs (1610, 1620, 1630, 1640), qui sont configurés pour détecter le faisceau laser qui a traversé la cellule de mesure en flux (1300).

6. Agencement de cytomètre en flux selon l'une quelconque des revendications précédentes, **caractérisé en ce que**
- l'agencement de cytomètre en flux (100) comprend un dispositif de commande (105), qui est configuré pour commander les pompes (310, 320).

7. Agencement de cytomètre en flux selon la revendication 6, **caractérisé en ce que**
- le dispositif de commande (105) est configuré pour commander la première pompe (310) et la deuxième pompe (320) de telle sorte qu'elles pompent simultanément des volumes prédéterminés d'échantillon liquide et de colorant dans le mélangeur (210).

8. Agencement de cytomètre en flux selon l'une quelconque des revendications 6 ou 7, **caractérisé en ce que**
- le dispositif de commande (105) est configuré pour commander la deuxième pompe (320) de telle sorte que la deuxième pompe (320) aspire le mélange à partir du mélangeur (210) ou de l'incubateur (220).

9. Agencement de cytomètre en flux selon la revendication 7 et la revendication 8, **caractérisé en ce que**
- le dispositif de commande (105) est configuré pour d'abord ordonner le pompage de l'échantillon liquide et du colorant dans le mélangeur (210), puis pour attendre un temps d'incubation prédéterminé, et ensuite pour ordonner l'aspiration du mélange.

10. Agencement de cytomètre en flux selon l'une quelconque des revendications 6 à 9, **caractérisé en ce que**
- le dispositif de commande (105) est configuré pour commander la première pompe (310) de telle sorte qu'elle pompe du liquide d'enveloppe dans la cellule de mesure en flux (1300), et pour commander simultanément la deuxième pompe (320) de telle sorte qu'elle pompe le mélange aspiré à partir du mélangeur (210) ou de l'incubateur (220) dans la cellule de mesure en flux (1300).
